# EUROPÄISCHE PATENTSCHRIFT

(11) **EP 1 007 740 B1**
(45) Veröffentlichungstag und Bekanntmachung des Hinweises auf die Patenterteilung: **14.10.2009**
(21) Anmeldenummer: 98946419.3
(22) Anmeldetag: 24.08.1998
(51) Int. Cl.: C12Q 1/68

(54) **VERFAHREN ZUR CHARAKTERISIERUNG DISSEMINIERTER UND MIKROMETASTASIERTER KREBSZELLEN**
METHOD FOR CHARACTERIZING DISSEMINATED AND MICROMETASTASIZED CANCER CELLS
PROCEDE PERMETTANT DE CARACTERISER DES CELLULES CANCEREUSES DISSEMINEES ET MICROMETASTASEES

(30) Priorität: 22.08.1997 DE 19736691
(43) Veröffentlichungstag der Anmeldung: 14.06.2000
(73) Patentinhaber: Giesing, Michael, 49536 Lienen (DE)
(72) Erfinder: GIESING, Michael, D-49536 Lienen (DE); AUSTRUP, Frank, D-45663 Recklinghausen (DE); DRIESEL, Gerhard, D-45663 Recklinghausen (DE); EDER, Claudine, D-45665 Recklinghausen (DE); FEIFEL, Nico, D-45657 Recklinghausen (DE); HOLEWA, Beatrix, D-45657 Recklinghausen (DE); SUCHY, Bernhard, D-45663 Recklinghausen (DE); UCIECHOWSKI, Peter, D-45665 Recklinghausen (DE)
(74) Vertreter: Kinzebach, Werner
(86) Internationale Anmeldenummer: PCT/EP1998/005360
(87) Internationale Veröffentlichungsnummer: WO 1999/010528

(56) Entgegenhaltungen:
- WO-A-94/10343
- WO-A-95/16792
- WO-A-96/17080
- WO-A-96/29430
- WO-A-96/35808
- WO-A-97/07242
- PANTEL K AND RIETHMÜLLER G: "Methods for detection of micrometastatic carcinoma cells in bone marrow, blood, and lymph nodes" ONKOLOGIE, Bd. 18, 1995, Seiten 394-401, XP002092612
- PELKEY J P ET AL.: "Molecular and immunological detection of circulating tumor cells and micrometastases from solid tumors" CLINICAL CHEMISTRY, Bd. 42, Nr. 9, 1996, Seiten 1369-1381, XP002092613
- NOGUCHI S ET AL.: "The detection of breast carcinoma micrometastasis in axillary lymph nodes by means of reverse transcriptase-polymerase chain reaction" CANCER, Bd. 74, Nr. 5, 1994, Seiten 1595-1600, XP002092614
- PITTMAN K ET AL.: "Reverse transcriptase-polymerase chain reaction for expression of tyrosinase to identify malignant melanoma cells in peripheral blood" ANNALS OF ONCOLOGY, Bd. 7, 1996, Seiten 297-301, XP002092615
- JUNG R ET AL.: "Quality management and influential factors for the detection of single metastatic cancer cells by reverse transcriptase polymerase chain reaction" EUROPEAN JOURNAL OF CLINICAL CHEMISTRY AND CLINICAL BIOCHEMISTRY, Bd. 35, Nr. 1, 1997, Seiten 3-10, XP002092616
- VAN DER VELDE-ZIMMERMANN D ET AL.: "Molecular test for the detection of tumor cells in blood and sentinel nodes of melanoma patients" AMERICAN JOURNAL OF PATHOLOGY, Bd. 149, Nr. 3, 1996, Seiten 759-764, XP002092617
- BURCHILL S A ET AL.: "Detection of epithelial cancer cells in peripheral blood by reverse transcriptase - polymerase chain reaction" BRITISH JOURNAL OF CANCER, Bd. 71, 1995, Seiten 278-281, XP002092618
- SMYTH M J ET AL.: "Antitumor activity of idarubicin-monoclonal antibody conjugates in a disseminated thymic lymphoma model" CANCER RESEARCH, Bd. 51, 1991, Seiten 310-317, XP002092619
- SCHMITZ-DRÄGER B J ET AL.: "Molecular biology of dissemination in bladder cancer - laboratory findings and clinical significance" WORLD JOURNAL OF UROLOGY, Bd. 14, 1996, Seiten 190-196, XP002092620

## Beschreibung

Die vorliegende Erfindung betrifft ein Verfahren zur

Charakterisierung disseminierter und mikrometastasierter Krebszellen mittels Nukleinsäureanalyse und die Verwendung dieses Verfahren zur in vitro-Diagnose von Krebs und zu weiteren Zwecken.

Die Diagnose von Krebs beim Menschen stellt nach wie vor eine der größten Nerausforderungen der heutigen Medizin dar. Gängige Diagnoseverfahren erlauben die Erkennung von krebsartigen Geschwülsten, die im folgenden umfassend als Tumore (Sarkome, Karzinome, systemisch-hämatologische Malignome) bezeichnet werden sollen, häufig erst dann, wenn der Tumor schon ein fortgeschrittenes Stadium erreicht hat. Trotz erheblicher Fortschritte bei bildgebenden Verfahren setzt deren erfolgreiche Anwendung immer eine gewisse Mindestgröße des Tumors voraus. Zudem läßt sich mit derartigen Verfahren - wenn überhaupt - nur sehr wenig weiterführende Information über die Art und Beschaffenheit des Tumors gewinnen. Die zeitaufwendigen und kostenintensiven bildgebenden Verfahren dienen daher in der Regel lediglich als Orientierungshilfen für das weitere, häufig direkte Vorgehen, in der Regel eine Gewebeentnahme. Letztere Maßnahme bedeutet aber einen invasiven Eingriff in den Körper des Patienten, der je nach Lage und Beschaffenheit des Tumors für den Patienten sehr unangenehm oder sogar gefährlich sein kann.

Wird anhand einer solchen Gewebeentnahme ein Tumor diagnostiziert, folgen normalerweise weitere Untersuchungen, die beispielsweise das Streuungspotential, d.h. die Metastasenbildung, dieses Tumors beschreiben sollen. Wird z.B.

Brustkrebs diagnostiziert, entnimmt man der betroffenen Patientin in der Regel 20 bis 30 Lymphknoten und bestimmt die Anzahl der Lymphknoten, die Krebszellen aufweisen. Die herrschende Meinung geht davon aus, daß die Überlebenschance einer Patientin mit zunehmender Anzahl befallener Lymphknoten abnimmt. Neuere Erkenntnisse deuten allerdings an, daß das Auftreten solcher Lymphknoten-Metastasen eher ein Maß für das Alter als für die Aggressivität oder das Metastasierungspotential des Tumors ist.

Im Zuge der fortschreitenden Entwicklung molekularbiologischer Methoden und dem zunehmenden Wissen über die genetischen Hintergründe der Zellentartung hat man in den letzten Jahren neue Wege beschritten, Krebs zu diagnostizieren. Man ging davon aus, daß Krebszellen charakteristische Marker exprimieren, aufgrund derer sie von nichtentarteten Zellen zu unterscheiden und somit als Krebszellen zu identifizieren sein sollten. Mit der Entwicklung der Hybridomtechnik und der dadurch ermöglichten Züchtung monoklonaler Antikörper wurde eine Vielzahl von Immunoassays entworfen, die durch den Nachweis bestimmter Marker zur Diagnose von Krebs beitragen sollen. Beispiele für solche Marker sind das karzinoembryonale Antigen (CEA), das α-Fetoprotein (AFP) oder das prostata-spezifische Antigen (PSA). In der EP 0 747 705 wurde kürzlich vorgeschlagen, nur die 90 kDa-Glycoform einer zu CEA strukturell verwandten Gruppe von Proteinen (NCAs) im Blut zu untersuchen, da scheinbar nur diese Glycoform in den Blutstrom abgegeben wird. Die WO 96/21862 gibt an, daß die Messung der Konzentration an A-Protein im Blut die Diagnose von Krebs gestatten würde, stellt aber auch fest, daß die Hoffnungen, die in bis dahin untersuchte Marker, wie CEA, AFP oder PSA gesetzt wurden, nicht erfüllt worden sind. Weitere auf dem immunologischen Nachweis bestimmter Proteine beruhende Verfahren werden beispielsweise in DE 195 00 723; WO 97/26271; WO 97/28186; WO 96/01907; US 5,633,142; US 5,620,848; US 5,589,579; und US 5,563,247 beschrieben. Eine verläßliche Diagnose von Krebs scheint allerdings auf diese Art zumindest zur Zeit nicht möglich zu sein.

Andere Forscherteams haben sich dagegen auf die Analyse des genetischen Materials von Krebszellen konzentiert. So schlägt die WO 93/04200 vor, zur Abschätzung einer Veranlagung für Brustkrebs DNA aus einer Blutprobe der Patientin zu isolieren, diese DNA auf bestimmte Weise zu restringieren und aufgrund des Restriktionsmusters eine entsprechende Diagnose zu stellen. Ein Nachweis von Tumorzellen im Blut ist mit dieser Methode allerdings nicht möglich.

Die WO 96/02671 beschreibt darüber hinaus ein Verfahren, mit dem man genomische DNA oder cDNA aus neoplastischem Gewebe, Blut oder einer anderen Körperflüssigkeit allein durch Sequenzanalyse (Sequenzierung oder Hybridisierung) auf Mutationen eines Gens untersucht, welches für ein Protein kodiert, dessen Funktionsstörung mit Krebs in Zusammenhang gebracht wird. Über ähnliche Ansätze wird in WO 97/26271; WO 97/28186; WO 96/15262; WO 96/01907; WO 93/22456; US 5,620,848; US 5,149,628 und WO 96/21021 berichtet.

Vielfach wird auch vorgeschlagen, die im Blutplasma frei vorhandene DNA und/oder RNA auf onkogene Mutationen oder Deletionen, Tumorsuppressor-Gen-Mutationen oder -Deletionen oder Veränderungen des Mikrosatellitenmusters zu untersuchen (vergleiche beispielsweise WO 95/16792; DE 37 21 400; DE 37 17 212; und WO 93/22456).

Schließlich beschreibt auch die WO 94/10343 ein Verfahren zur Diagnose von Krebs, nämlich zur Erkennung von Prostatakrebs-Mikrometastasen, wobei man das Blut eines Patienten auf RNA untersucht, die für das prostataspezifische Antigen kodiert. Allerdings werden bei diesem Verfahren in etwa zwei Dritteln der Fälle sogenannte falsch-positive Antworten erhalten, da auch Entzündungen oder Verletzungen der Prostatadrüse die Konzentration des Proteins im Blut erhöhen. Andererseits bleiben aufgrund von Unzulänglichkeiten des Tests vermutlich 30 % der Krebserkrankungen nach wie vor unentdeckt. Eine **ähnliche Vorgehensweise wird in** US 5,601,990 **zur Diagnose von metastasierendem Darmkrebs gewählt.** WO 96/35808 beschäftigt sich mit dem Nachweis eines Proteins (hCRG). WO 96/17080 beschreibt die Detektion von Tumoren und/oder deren Metastasierung durch den Nachweis von CK20. Das in Noguchi, S. et al., Cancer, Band 74, Nr. 5,1994, Seiten 1595-1600, beschriebene Verfahren basiert auf der Amplifikation von MUC1-mRNA. Pittman, K. et al., Annals of Oncology, Band 7,1996, Seiten 297-301 und van der Velde-Zimmermann, D. et al., American Journal of Pathology, Band 149, Nr. 3,1996, Seiten 759-764, greifen zum Nachweis von zirkulierenden Melanomzellen auf die Tyrosinase-mRNA zurück. In Burchill, S. A. et al., British Journal of Cancer, Band 71, 1995, Seiten 278-281, wird ausgeführt, CK20-mRNA könne möglicherweise zum Nachweis metastasierender Tumorzellen epithelialen Ursprungs dienen, während CK8 und CK19 dazu nicht geeignet seien. WO 97/07242 behandelt immunologische Verfahren und WO 97/07242 beschreibt eine RT-PCR auf PSA.

In Pantel, K. und Riethmüller, G., Onkologie, Band 18,1995, Seiten 394-401, wird Stellung genommen zu mutationsspezifischen PCR-Analysen (Ki-ras und p53) sowie zu RT-PCR-Ansätzen zum Nachweis so genannter gewebsspezifischer Antigene (CK19, CEA, PSA, PSM). Während die mutationsspezifischen Analysen auf Primärtumore angewandt werden, sollen die RT-PCR-Analysen auch an Blutproben eingesetzt werden. Allerdings wird jeder der in Betracht gezogenen epithelialspezifischen mRNA-Marker für sich genommen und ohne Bezug zu anderen besprochen. Jung, R. et al., European Journal of Clinical Chemistry and Clinical Biochemistry, Band 35, Nr. 1, 1997, Seiten 3-10, behandelt verschiedene Originalarbeiten, denen jeweils einzelne RT-PCR-Analysen zugrunde liegen. Schmitz-Dräger, B. J. et al., World Journal of Urology, Band 14,1996, Seiten 190-196, fasst verschiedene Ansätze verschiedener anderer Arbeitsgruppen zusammen, die Primärtumore analysieren und daraus metastasierungsrelevante Faktoren ableiten.

WO 96/29430 beschreibt ein als "Multiple Marker Assay" bezeichnetes Verfahren zum Nachweis von Melanom- oder Brustkrebsmetastasen, mit dem mehr als 1 Parameter untersucht wird. Dazu wird allerdings ausschließlich die RT-PCR-Methodik zum Nachweis von mRNAs eingesetzt, die in der untersuchten Körperflüssigkeit normalerweise nicht vorkommen. So wird beispielsweise der Buffy-Coat aus Blut gewonnen und entsprechend untersucht. Pelkey, J. P. et al., Clinical Chemistry, Band 42,1996, Seiten 1369-1381, soweit über die Einzelparameter-Analytik hinausgehend, besprechen lediglich die der WO 96/29430 zugrunde liegende Arbeit.

Zusammenfassend läßt sich daher feststellen, daß der Stand der Technik keine zuverlässige Diagnose von Krebs zuläßt.

Der vorliegenden Erfindung liegt daher die Aufgabe zugrunde, ein in-vitro Verfahren zu schaffen, mit dem alle Krebsformen von Säugern und insbesondere von Menschen zuverlässig und hinreichend genau, auch für einzelne Patienten, erkannt und beurteilt werden können, und das es ermöglicht, den Verlauf einer geeigneten Krebstherapie zu verfolgen. Das Verfahren soll außerdem das Testen von Wirkstoffen auf antineoplastische Wirkung ermöglichen.

Diese Aufgabe wird erfindungsgemäß durch ein Verfahren zur Charakterisierung disseminierter und mikrometastasierter Krebszellen mittels Nukleinsäureanalytik gelöst, wobei man
i) aus Körperflüssigkeit eines Individuums gewonnene Zellen auf wenigstens eine mRNA eines krebsspezifischen ersten Gens untersucht; und
ii) aus Körperflüssigkeit eines Individuums abgetrennte Krebszellen auf wenigstens eine DNA oder mRNA eines krebsspezifischen oder krebsassoziierten zweiten Gens untersucht und die gleiche Untersuchung mit Nichtkrebszellen desselben Individuums zum Vergleich durchführt,
wobei das erste und das zweite Gen verschieden sind.

Unter Charakterisierung werden erfindungsgemäß alle jene Maßnahmen verstanden, die an Zellen von Säugern und insbesondere von Menschen durchgeführt werden können, um ein, zwei, drei, vier, fünf, 6 bis 10, 11 bis 20 oder mehr krebsspezifische und/oder krebsassoziierte Gene dieser Zellen qualitativ oder quantitativ zu erfassen. Aufgrund der durch die Charakterisierung gewonnenen Ergebnisse kann dann die Identifizierung der Zellen vorgenommen werden. Dies umfaßt erfindungsgemäß nicht nur den qualitativen Nachweis von zirkulierenden Krebszellen, sondern kann auch deren Quantifizierung und/oder Angaben über deren Herkunft und Verhalten, beispielsweise bezüglich der Metastasenbildung oder bei verschiedensten, beispielsweise cytotoxischen Therapieansätzen beinhalten.

Zu zirkulierenden Krebszellen gehören erfindungsgemäß vor allem solche Krebszellen, die sich vom Primärtumor abgelöst haben, d.h. disseminierte und mikrometastasierte Krebszellen. Im folgenden wird zwecks Vereinfachung von zirkulierenden Krebszellen gesprochen. Da die Streuung dieser Zellen in der Regel mit der Vaskularisierung des Primärtumors zusammenhängt, kann man zirkulierende Krebszellen insbesondere im Blut auffinden, wobei auch Knochenmark und Lymphknoten geeignet sind. Dementsprechend werden erfindungsgemäß Körperflüssigkeiten, wie Blut, Lymphe, Urin, Knochenmark und verschiedene Organspülflüssigkeiten wie Bronchiallavage, Pankreas- oder Blasenspülflüssigkeit untersucht.

Erfindungsgemäß wird zwischen krebsspezifischen und krebsassoziierten Genen unterschieden. Krebsspezifisch im erfindungsgemäßen Sinn sind solche Gene, anhand derer sich eine zirkulierende Krebszelle als solche erkennen läßt. Krebsassoziierte Gene dagegen sind nicht spezifisch für Krebszellen. Sie können auch in gesunden Zellen oder bei einer Vielzahl verschiedener anderer Erkrankungen, beispielsweise Entzündungen, exprimiert werden. Allerdings kann deren Expression in Krebszellen im Vergleich zu Nichtkrebszellen charakteristisch moduliert sein, so daß weitere Rückschlüsse auf die Art und das Verhalten der Krebszellen möglich sind.

In diesem Sinn ist es natürlich auch möglich, daß ein bestimmtes Gen sowohl zur krebsspezifischen als auch krebsassoziierten Charakterisierung beitragen kann. Beispielsweise kann ein Gen eine Mutation aufweisen, die zur anomalen Expression eines Zellzyklus-regulierenden Proteins und infolgedessen zur Entartung der betroffenen Zelle führt. Dieses mutierte Gen ist daher krebsspezifisch und die erfindungsgemäße Untersuchung auf den Nachweis dieses mutierten Gens dient zur krebsspezifischen Charakterisierung. Darüber hinaus kann eine Analyse der anomalen Expression dieses Gens auch zur krebsassoziierten Charakterisierung beitragen, da die Art oder Menge entsprechender Expressionsprodukte für den Zellzyklus von Bedeutung sind und dadurch weitere Rückschlüsse auf die Art und das Verhalten der Krebszellen möglich sind.

Die Untersuchung auf krebsspezifische und krebsassoziierte Gene kann auf jede dem Fachmann erdenkliche Art und Weise vorgenommen werden. So kann ein erfindungsgemäßes Gen auf DNA-Ebene, auf RNA-Ebene und/oder auf Proteinebene untersucht werden. Auf DNA-Ebene untersucht man vorzugsweise genomische DNA auf Mutationen, Amplifikationen, LOH's, Translokationen und/oder Polymorphismen. Auf RNA-Ebene und auf Proteinebene untersucht man Expressionsprodukte, nämlich vorzugsweise Transkriptionsprodukte wie mRNA bzw. Translationsprodukte wie Proteine. Bevorzugt sind Methoden, mit deren Hilfe die Beteiligung eines Gens am Zustand zirkulierender Zellen zum Untersuchungszeitpunkt bewertet werden kann, z.B. die Untersuchung von mRNA insbesondere im Hinblick auf die in einer Zelle vorhandene Menge an einer bestimmten mRNA. Beinhaltet das erfindungsgemäße Verfahren eine Untersuchung einer Körperflüssigkeit auf Proteine, so handelt es sich insbesondere um diejenigen, die von den krebsspezifischen und/oder krebsassoziierten Genen exprimiert werden. Im folgenden wird, sofern nichts anderes angegeben ist, umfassend von einer Untersuchung oder Analyse der erfindungsgemäßen Gene gesprochen.

Die in der nachfolgenden Beschreibung aufgeführten spezifischen Gene werden häufig mit Abkürzungen oder Codes bezeichnet, die üblicherweise verwendet werden und dem Fachmann deshalb bekannt sind. Darüber hinaus kann das am Ende der vorliegenden Beschreibung eingefügte Glossar zur Erläuterung herangezogen werden.

Zu den erfindungsgemäßen krebsspezifischen Genen zählen insbesondere zwei Klassen von Genen, die bei der Krebsentstehung eine wesentliche Rolle spielen: Onkogene, die durch Mutation aus sogenannten Proto-Onkogenen entstehen und mutierte Tumorsuppressor-Gene. Beide dirigieren in ihrer normalen Form den Lebenszyklus einer Zelle: Proto-Onkogene fördern das Zellwachstum, Tumorsuppressor-Gene bremsen es. Onkogene sind krebabegünstigend, da sie die Zelle zu übermäßiger Vermehrung anregen, während Tumorsuppressor-Gene dann zur Krebsentstehung beitragen, wenn sie durch Mutation inaktiviert werden und die Zelle infolgedessen eine Wachstumsbremse verliert, durch die sie normalerweise an einer unangemessenen Vermehrung gehindert wird. Onkogene kodieren beispielsweise für Wachstumsfaktoren und deren Rezeptoren, Signalproteine, Transkriptionsfaktoren und eine Vielzahl anderer Proteine, von denen einige beispielsweise bei der Apoptose eine wichtige Rolle spielen. Zu den Onkogenen zählen beispielsweise Gene, wie die bcl-2-Familie, mdm2, c-abl, die myc-Familie, beispielsweise c-, N-, R-, L- und B-myc, die ras-Familie, beispielsweise H-, K- und N-ras, erb-B2, das auch neu genannt wird, erb-B, PDGF, RET und virale Onkogene verschiedener Tumorviren, wie Papova-Viren, beispielsweise SV40-, Polyoma- und Papilloma-Viren, wie HPV, Adenoviren, bestimmte Herpesviren, Pockenviren, Hepatitis-B-Viren (HBx-Gen), Hepatitis-C-Viren, HTLV-1, E1A-Fusionstranskript bei Adenoviren, E6- und E7-Fusiönstranskripte bei HPV und EBV beim Burkitt-Lymphom.

Erfindungsgemäß bevorzugte Onkogene sind Gene der ras-Familie, erb-B2, erb-B, c-myc, mdm2, bcl-2, Hepatitis-B-Viren (HBx-Gen), Hepatitis-C-Viren, HTLV1, E1A-Fusionstranskript bei Adenoviren, E6- und E7-Fusionstranskripte bei HPV und EBV beim Burkitt-Lymphom. Ganz besonders bevorzugt sind c-myc, k-ras und erb-B2.

Zu den Tumorsuppressor-Genen zählen beispielsweise die Gene der APC-Familie (FAP), DCC, DPC4, NF-1, NF-2, MTS1, RB, p53, WT1, BRCA1, BRCA2, VHL, MSH2, MLH1 und WAF1.

Erfindungsgemäß bevorzugte Tumorsuppressor-Gene sind p53, RB, APC, DCC, BRCA1, BRCA2, MSH2, MLH1 und WAF1. Ganz besonders bevorzugt sind p53, RB, APC, DCC und DPC4.

Zu den krebsspezifischen Genen zählen auch die neben Onkogenen und mutierten Tumorsuppressor-Genen auch Gene, die bei Nichtkrebszellen in erfindungsgemäß untersuchten Körperflüssigkeiten abgeschaltet sind, d.h. nicht oder nur in unwesentlichen Mengen exprimiert werden. Werden daher Transkriptions- und/oder Translationsprodukte dieser Gene in einer Körperflüssigkeit, beispielsweise Blut, nachgewiesen, spricht dies für das Vorliegen zirkulierender Krebszellen in der betreffenden Körperflüssigkeit.

Hierzu zählen beispielsweise hCG, hTG, Calcitonin, Albumin, Surfactant-Proteine, Telomerase, verschiedene Trans-lokationen, Stat5a, Varianten von Steroidrezeptoren (ÖR, AR), Progesteron-Rezeptor, verschiedene Gene, die ein LOH aufzeigen, CEA, PSM, PSA, AFP, Tyrosinase, MAGE3, Mucl8, MUC1, Cytokeratine, insbesondere CK20 und CK19, LOH-Untersuchungen in verschiedenen Chromosomenabschnitten durch zahlreiche Mikrosatelliten, Magen-Darm-Trakt-Hormone, wie Motilin, Enteroglucagon, GIP, Gastrin, CCK oder PYY, und Neurotensin. Erfindungsgemäß bevorzugt sind CEA, PSM, MUC1 (tumorspezifische Splice-Variante), AFP, Cytokeratin, Tyrosinase, MAGE3, MUC18, tumorspezifische Splice-Varianten des Östrogen- und Androgen-Rezeptors, und EGP.

Zu derartigen Genen zählen auch die unten aufgeführten gewebsspezifischen Gene, die aufgrund ihrer Gewebsspezifität zur erfindungsgemäßen krebsassoziierten Charakterisierung beitragen, aber durch die Eigenart des Untersuchungsobjektes, nämlich in einer Körperflüssigkeit zirkulierender, vom Primärtumor losgelöster Krebszellen, auch zur krebsspezifischen Charakterisierung herangezogen werden können.

Darüber hinaus können auch prognostische Onkoproteine, wie anti-p53, pan p53, p53 oder c-erb-B2, zur krebsspezifischen Untersuchung herangezogen werden.

Gemäß einer bevorzugten Ausführungsform der vorliegenden Erfindung untersucht man eine Körperflüssigkeit auf wenigstens ein krebsspezifisches Gen und wenigstens ein krebsassoziiertes Gen. Dazu untersucht man aus Körperflüssigkeit eines Individuums gewonnene Zellen zusätzlich auf wenigstens ein krebsassoziiertes Gen, das bei Nichtkrebszellen der untersuchten Körperflüssigkeit im wesentlichen nicht exprimiert wird; und/oder untersucht man aus Körperflüssigkeit eines Individuums abgetrennte Krebszellen zusätzlich auf wenigstens ein krebsassoziiertes Gen und führt die gleiche Untersuchung mit Nichtkrebszellen desselben Individuums zum Vergleich durch.

Die erfindungsgemäßen krebsassoziierten Gene umfassen ein breites Funktionsspektrum. Insbesondere geeignet sind gewebsspezifische, d.h. organotypische Gene (Morphogene), die Aussagen über die Herkunft der zirkulierenden Krebszellen zulassen, so daß auf die Lokalisation des Primärtumors, der Streuquelle, geschlossen werden kann; Gene, welche die Metastasierungsfähigkeit der Krebszellen kennzeichnen; Gene, die für Steroidhormonrezeptoren kodieren, so daß Aussagen über den Rezeptorstatus der Krebszellen möglich sind; Chemoresistenz-Gene; oder Gene, deren Expression mit der Modulierung der Immunantwort sowie der Zellproliferation und Apoptose zirkulierender Krebszellen korreliert. Die Expression dieser krebsassoziierten Gene kann bei Krebszellen in charakteristischer Weise moduliert sein, so daß das resultierende Expressionsmuster auch einen Hinweis auf Krebs geben kann.

Krebsassoziierte gewebsspezifische Gene kodieren häufig für organotypische Marker, also Proteine bzw. Antigene, aufgrund derer sich auf die Herkunft der das Gen exprimierenden Zelle schließen läßt. Hierzu zählen beispielsweise Leberspezifische Gene, wie Albumin oder AFP; Prostata-spezifische, wie AR, PSM, hK2 oder PSA; Mamma-, Ovar- und/oder Cervixspezifische, wie β-hCG, ÖR, PR, SCCA-1, Maspin oder BA46; colorectal-spezifische, wie CCK, Enteroglucagon, GIP, Gastrin, Motilin oder PYY; Pankreas-spezifische wie PYY; Melanom-spezifische, wie MAGE1, MAGE3, Muc18 oder Tyrosinase; Schilddrüsen-spezifische, wie hTG; Lungen-spezifische, wie SF, SF-R, Surfactant-Proteine, beispielsweise SP-A und SP-C, CC10, N-CoR oder RARβ2; Blasen-spezifische, wie EGF-R und β-hCG; Endometrium-spezifische wie SCCA.

Zur gewebsspezifischen Charakterisierung kann auch auf Onkogene und/oder mutierte Tumorsuppressor-Gene zurückgegriffen werden, wenn sie auf bestimmte Formen von Krebs hinweisen. Beispiele hierfür sind tumorassoziierte Mutationen, wie Translokation 14;18 (bcl-2) für Lymphome, Translokation 9;22 (BCR/ABL) für chronische myeloische Leukämien, Translokation 15;11 für akute nichtlymphozytäre Leukämien, Translokation 2;13 (PAX3-FKHR) und Translokation 1;13 (PAX7-FKHR) für alveolare Rhabdomyosarkome, Translokation 11;22 für Ewing Sarkome, Translokation 12;16 für myoxoide Liposarkome, Translokation x;18 für Synovialsarkome; BRCA-1 und BRCA-2 für Mammakarzinome, DPC-4 für Pankreaskarzinome, erb-B für Glioblastome, MLH-1 und MSH-2 für HNPCC (hereditary nonpolyposis colon cancer), NF-2 für Neurofibromatose-1, NF-1 für Neurofibromatose, RET für Schilddrüsenkarzinome, RB für Retinoblastome, VHL für Nierenkarzinome, WT-1 für Nierentumoren, und k-ras für Colonkarzinome.

Die Charakterisierung der Metastasierungsfähigkeit zirkulierender Krebszellen nimmt erfindungsgemäß eine besondere Stellung ein. Zu diesem Zweck werden die Zellen insbesondere auf Gene untersucht, die für Angiogenese-, Wachstums- und Motilitätsfaktoren, Matrixdegradationsfaktoren, wie Proteasen und deren Hemmstoffe, oder Adhäsionsfaktoren, wie Adhaerine, kodieren.

Zu den Angiogenesefaktoren zählen beispielsweise aFGF und bFGF sowie deren Rezeptoren aFGF-R und bFGF-R, VEGF und dessen Rezeptoren VEGF-R1 und VEGF-R2, sowie GD-AIF.

Zu den Wachstumsfaktoren zählen beispielsweise TGF-α und TGF-β, IGF, IGF-BP3, erb-B (EGF-R), PDGF und EGF.

Zu den migrationstimulierenden Motilitätsfaktoren zählen beispielsweise der Scatter-Faktor SF-L und dessen Rezeptor SF-R (c-met).

Die Proteasen und deren Hemmstoffe umfassen beispielsweise Matrix-Hydrolasen, wie MMP's (Matrix-Metalloproteasen), MT-MMP, UPA (Urokinase-artiger Plasminogenaktivator) oder deren Inhibitoren, wie PAI1 und PAI2 (Plasminogenaktivator-Inhibitor) oder TIMP's (Gewebeinhibitoren von Metalloproteasen).

Zu den Adhaerinen zählen Adhäsionsproteine, wie Cadhaerine, beispielsweise E-Cadhaerin, Catenine, beispielsweise β-Catenin, Selectine, beispielsweise E-, P- und L-Selectin sowie deren Rezeptoren, CD44 (Standard- und Splice-Varianten), Integrine und ICAM's.

Erfindungsgemäß bevorzugte Gene zur Charakterisierung der Metastasierungsfähigkeit sind Angiogenesefaktoren (bFGF und bFGF-R; VEGF und VEGF-R's), Proteasen (UPA; PAI; MMP's; TIMP's), Adhaerine (E-Cadherin; α-Catenin; β-Catenin; Selectin-L und -R; CD44), Motilitätsfaktoren SF-L und c-met und Metastasen-Suppressor nm23.

Von den Steroidhormonrezeptor-Genen kommen erfindungsgemäß bevorzugt die Gene zur Anwendung, die für den Östrogen-, Progesteron- oder Androgen-Rezeptor (ÖR, PR oder AR) kodieren.

Auch die Charakterisierung von Chemoresistenz-Genen zirkulierender Krebszellen ist erfindungsgemäß von besonderer Bedeutung, da Krebszellen häufig gegen Therapeutika, zum Teil auch mehrfach, resistent sind und eine Charakterisierung dieser Gene zur Bewertung der Erfolgschancen bestimmter Krebstherapien beitragen kann, Beispiele für solche Chemoresistenz-Gene sind MDR1, das für das P-Glykoprotein kodiert, nm23, hMLH1, gp170, MRP1, das Topoisomerase-Gen, das Glutathion-S-Transferase-pi-Gen, das LRP-Gen und Gene, die für α- oder β-Tubulin kodieren.

Erfindungsgemäß bevorzugt untersuchte Chemoresistenz-Gene sind MDR1, MRP1, das Topoisomerase II-Gen, das LRP-Gen, das β-Tubulin-Gen und das Glutathion-S-Transferase-pi-Gen.

Zur Charakterisierung einer Modulierung der Immunantwort kann man sich beispielsweise der Bewertung der T- und NK-zellvermittelten Cytotoxizität und/oder antikörperabhängigen zellvermittelten Cytotoxizität (ADCC) bedienen. Zu diesem Zweck kann man immunologische Effektorzellen, insbesondere NK-, H1/TH2- und CD8-Zellen auf der einen Seite und zirkulierende Krebszellen auf der anderen Seite beispielsweise auf das TNF-α-Gen (Tumornekrose-Faktor), Gene, die für Interferone, beispielsweise α- und γ-IFN kodieren, FAS-Liganden- und FAS-Rezeptoren-Gene, Perforini, bcl-2, bax und Granzyme untersuchen. Bevorzugt sind FAS-R und FAS-L, Perforin und Granzyme.

Die Proliferations- und Apoptoseeigenschaften zirkulierender Krebszellen werden erfindungsgemäß anhand von Genen untersucht, die mit dem Proliferations- und Apoptosestatus von Zellen, insbesondere von Krebszellen, korrelieren. Dazu zählen unter anderen auch einige der oben genannten Onkogene bzw. Proto-Onkögene und Tumorsuppressor- bzw. mutierten Tumorsuppressor-Gene, die - über die krebsspezifische Charakterisierung hinaus - aufgrund ihres Expressionsmusters auch zur krebsassoziierten Charakterisierung beitragen können. Erfindungsgemäß wird beispielsweise auf p53, durch p53 sequenzspezifisch transkriptional aktivierte (Bax; FAS-L und -R; Cycline A, B1, D1, D2, D3, E oder G; GADD45; GD-AIF; HIC1; IGF-BP3; mdm2; p21) und inaktivierte Gene (bcl-2; c-myc; bFGF, c-fos; HSP70; IL-6; MDR1; PCNA), zu Beginn der Apoptose und des Zellzyklusarrestes exprimierte Gene (außer p53 noch TNF-α, TNF-R1, TNF-R2, DPC-4, IFN-y und FAS-L und - R) sowie Gene, die bei unreguliertem Wachstum vorkommen, wie erb-B2, EGF und sonstige autokrine Wachstumsfaktoren (TGF-α; PDGF) zurückgegriffen. Bevorzugt sind Bax, FAS, Cycline, mdm2, p21, p16, bcl-2, c-myc, FGF, MDR1, TNF-α, IFN-y, erb-B2, EGF und sonstige autokrine Wachstumsfaktoren.

Zur tumorbiologischen Untersuchung, d.h. zur Charakterisierung einer Modulierung der Immunantwort, der Proliferations- und Apoptoseeigenschaften, werden Cycline, insbesondere die Cycline B1, D1 und E, Ki67, FAS-L, FAS-R, bax und/oder bcl-2 bevorzugt untersucht.

Die erfindungsgemäße Untersuchung von Körperflüssigkeiten erfolgt mittels Nukleinsäureanalyse. Hierzu gehören beispielsweise Untersuchungen von DNA oder RNA, insbesondere mRNA, mit Hilfe von Techniken, die dem Fachmann bekannt sind, wie Sequenzierungstechniken, Hybridisierungstechniken, beispielsweise Northern- oder Southern-Blotting, Hybridisierung auf Microchips, insbesondere Verfahren, die auf der Polymerasekettenreaktion (PCR) beruhen und auch Techniken, bei denen die zu untersuchende DNA oder RNA zunächst in-vitro transkribiert und/oder translatiert wird. Erfindungsgemäß kann jede Körperflüssigkeit mit Hilfe einer Nukleinsäureanalyse untersucht werden. Vorteilhafterweise greift man auf Blut zurück, insbesondere dann, wenn mRNA untersucht wird. Zur Untersuchung eines Gens kann natürlich auch eine Kombination aus unterschiedlichen Nukleinsäureanalysen eingesetzt werden.

Die Untersuchung einer Körperflüssigkeit kann durch Anwendung bekannter immunologischer Verfahren vorgenommen werden. Hierzu zählen beispielsweise Immunopräzipitations- und Kompetitionsexperimente, Immunofluoreszenz, immunohistochemische Färbeverfahren, Western-Blotting, Durchflußcytometrie, ELISA u.ä. sowie massenspektrometrische Verfahren. Da immunologische Verfahren in der Regel auf bestimmte Antigän-Antikörper-Wechselwirkungen abstellen, dienen solche Verfahren vorzugsweise zur Untersuchung der Körperflüssigkeit auf Proteine, wobei es sich insbesondere um Proteine handelt, die von den vorstehend beschriebenen Genen exprimiert werden. Zu diesem Zweck eventuell benötigte Antikörper sind dem Fachmann entweder bekannt oder können nach üblichen Verfahren erhalten werden. Erfindungsgemäß werden immunologische Verfahren bevorzugt zur Untersuchung von Blut und insbesondere von Knochenmark eingesetzt. Mit immunologischen Verfahren lassen sich vorteilhafterweise z.B. folgende Proteine analysieren: P53, ERB-B2 und Tumorantigene mittels ELISA und ähnlicher Verfahren; FAS-Ligand und FAS-Rezeptor, Phosphatidylserin, Cytokine, Perforin, Cytokeratine und Cycline mit Hilfe von Immunphänotypisierung.

Auch eine Kombination aus immunologischen Verfahren und Nukleinsäureanalysen kann von Vorteil sein.

Viele der krebsspezifischen und krebsassoziierten Gene werden vorzugsweise anhand von mRNA untersucht. Eine zu diesem Zweck angewandte Technik ist die direkte Hybridisierung von mRNA und/oder cDNA (qualitativ/quantitativ) auf einer soliden Matrix (z.B. in Form von Microchips) mit immobilisierten Oligonucleotiden bzw. immobilisiertem Streptavidin und biotinylierten Oligonukleotiden. Auf diese werden mRNA, cDNA oder doppelsträngige PCR-Produkte hybridisiert. Für die Einführung des Signals stehen verschiedene Wege zur Verfügung, z.B. Primerextension durch markierte dNTP und ddNTP. Entsprechend der Markierung wird das Detektions-Prinzip gewählt: Radioaktivität, Fluoreszenz, Chemilumineszenz oder weitere dem Fachmann zu diesem Zweck bekannte Methoden.

Insbesondere bedient man sich einer bekannten Technik, welche die reverse Transkription (RT) und die Polymerasekettenreaktion (PCR) kombiniert und im folgenden als RT-PCR bezeichnet wird. Bei diesem Verfahren wird zunächst die mRNA aus den Zellen einer erfindungsgemäßen Körperflüssigkeit isoliert. Diese wird dann mit Hilfe der reversen Transkriptase zu cDNA umgeschrieben, welche in Folge mit Hilfe der PCR amplifiziert wird. Die so erhaltenen PCR-Produkte können dann entweder einer Fragmentanalyse, ggf. nach geeigneter Aufreinigung, zugeführt, direkt oder indirekt über weitere Klonierungszyklen sequenziert oder auch in-vitro exprimiert werden. Die Quantifizierung der untersuchten mRNA's erfolgt über verschiedene interne Kontrollen, vorzugsweise in Form von Zelläquivalenten oder klonierten cDNA's bzw cRNA's durch fluoreszenzmarkierte Primer, durch "real-time"-PCR oder durch RNA-Hybridisierung auf Microchips. Die zellspezifische Quantifizierung von Genen erfolgt über interne Standards, insbesondere vom Zelltyp unabhängige RNA (cDNA), die z.B. für GAPDH, β-Mikroglobulin, L32 oder β-Actin kodiert. Die Spezifität wird durch umfangreiche Kontrollen abgesichert, wie Mismatch-Proben oder die Sequenzierung der cDNA.

Bei den krebsspezifischen Genen, die bevorzugt anhand von mRNA-Analysen an Körperflüssigkeiten charakterisiert werden, handelt es sich insbesondere um die oben beschriebenen Gene, die bei Nichtkrebszellen in der untersuchten Körperflüssigkeit im wesentlichen nicht exprimiert werden.

Krebsassoziierte Gene werden in der Regel bevorzugt anhand von mRNA-Analysen an Körperflüssigkeiten charakterisiert. Hierzu zählen insbesondere folgende Gene: bFGF, bFGF-R, VEGF, VEGF-R's, MMP's, TIMP's, MDR1, MRP, LRP, Topoisomerase II, Gluta-thion-S-Transferase, Progesteron-Rezeptor, Bax, bcl-2, FAS-L, FAS-R, mdm2, p21, p16, c-myc, TNF-α, IFN-y, erb-B2 und EGF.

Eine DNA-Analyse, insbesondere die Sequenzanalyse genomischer DNA, ist in der Regel für die Untersuchung auf Onkogene und/oder mutierte Tumorsuppressor-Gene bevorzugt und kann insbesondere bei der Charakterisierung folgender Gene von Vorteil sein: p53, ras-Familie, erb-B2, c-myc, mdm2, BRCA1, BRCA2, APC, DCC, RB MSH2, MLH1, RET und LOH-Untersuchungen in verschiedenen Chromosomenabschnitten durch zahlreiche Mikrosatelliten.

Körperflüssigkeiten können in dem Zustand analysiert werden, in dem sie gewonnen wurden. Allerdings werden die Proben erfindungsgemäß in der Regel zunächst durch in an sich bekannte Maßnahmen auf die nachfolgende Untersuchung vorbereitet, indem Zellen bzw. zellhaltige Konzentrate oder zellhaltige Flüssigkeiten aus der Körperflüssigkeit gewonnen werden. Dies gilt insbesondere für die Nukleinsäureanalysen. So können beispielsweise anstelle von Blut bestimmte davon abgeleitete zellhaltige Flüssigkeiten oder Zellkonzentrate, beispielsweise der sogenannte Buffy-Coat oder Zellfraktionen nach Dichtezentrifugation, vorteilhafterweise verwendet werden. Die aus Körperflüssigkeit gewonnenen Zellen können dann insbesondere auf solche Gene untersucht werden, die bei Nichtkrebszellen der untersuchten Körperflüssigkeit im wesentlichen nicht exprimiert werden.

Bei der Untersuchung auf Gene, die auch von Nichtkrebszellen in der untersuchten Körperflüssigkeit exprimiert werden oder bei Untersuchungen auf genomische DNA untersucht man erfindungsgemäß in der Regel aus der Körperflüssigkeit abgetrennte Krebszellen.

Zur Abtrennung von Krebszellen können bekannte Verfahren eingesetzt werden, beispielsweise physikalische Verfahren wie Mikro-Filtration oder Dichtegradientenzentrifugation, oder antigenspezifische Immunadsorptionsverfahren, bei denen spezifische Antikörper die Krebszellen derart markieren, daß sie anschließend aussortiert werden können. Geeignete Antikörper (z.B. anti-EGP) sind beispielsweise mit fluoreszierenden und insbesondere magnetischen Markern versehen, so daß bei Verwendung eines derart markierten krebszellspezifischen Antikörpers Krebszellen nach Bindung solcher Antikörper in sogenannten Zellsortern isoliert werden können. Zur Auswahl geeigneter Antikörper zu Isolierungszwecken bestimmter Krebszellen kann man auf die Charakterisierung und Identifizierung dieser Krebszellen ohne vorherige Isolierung zurückgreifen. Vorzugsweise werden die Krebszellen in lebensfähigem und insbesondere vermehrungsfähigem Zustand isoliert. Insbesondere die mRNA sollte für die oben beschriebenen Untersuchungen intakt sein.

Die nach einer Separation erhaltenen Zellfraktionen müssen dann gegen Zelltyp-unabhängige Marker (beispielsweise GAPDH, β₂-Mikroglobulin, L32 oder β-Actin) quantifiziert werden. Ein weiterer Reinheitsnachweis kann durch RNA erfolgen, die für MNCs (mononukleäre Zellen) spezifisch ist (Perforin, CD45). Die nach Separation erhaltenen verschiedenen Zellfraktionen (Fraktion A: MNC einschließlich der Tumorzellen; Fraktion B: MNC nach der Entfernung der Tumorzellen; Fraktion C: aufgereinigte Tumorzellen) werden dann auch miteinander verglichen, indem man die abgetrennten Krebszellen untersucht und die gleiche Untersuchung mit Nichtkrebszellen desselben Individuums zum Vergleich durchführt. Dadurch werden patienteneigene Kontrollen in die Untersuchung miteinbezogen.

Gemäß einer besonderen Ausführungsform der vorliegenden Erfindung trennt man einzelne Krebszellen aus der Körperflüssigkeit ab und untersucht diese auch einzeln. Zu diesem Zweck kann durch eine sogenannte Einzelzell-PCR durch Genomamplifikation ein verändertes Genom einer einzelnen entarteten Zelle analysiert werden.

Die Isolierung zirkulierender Krebszellen wird vorteilhafterweise für genomische Untersuchungen und die Untersuchung auf Gene durchgeführt, die auch von Nichtkrebszellen in der untersuchten Körperflüssigkeit exprimiert werden, z.B. folgenden Genen:
DNA: p53, ras-Familie, erb-B2, c-myc, mdm2, RB, APC, DCC, LOH-Untersuchungen in verschiedenen Chromosomenabschnitten durch zahlreiche Mikrosatelliten:
RNA: bFGF, bFGF-R, VEGF-R's, MMP's, TIMP's, MDR1, MRP, LRP, Topoisomerase, Glutathion-S-Transferase, Bax, bcl-2, FAS, mdm2, p21, p16, c-myc, FGF, MDR1, TNF-α, IFN-y und EGF, AR, ÖR, EGP und SF.

Bestimmte erfindungsgemäße Untersuchungen werden bevorzugt an Zellkulturen vorgenommen. Dazu kann man die zirkulierenden Krebszellen in der oben beschriebenen Weise isolieren und anschließend unter geeigneten Bedingungen kultivieren. Insbesondere Aussagen zur Tumorbiologie (z.B. der Modulierung der Immunantwort durch Krebszellen oder der Proliferation) dieser Zellen lassen sich an in-vitro Kulturen treffen.

Gene, die vorteilhafterweise an in-vitro kultivierten Krebszellen charakterisiert werden, sind z.B. folgende: Bax, FAS, Cycline, mdm2, p21, p16, bcl-2, c-myc, FGF, MDR1, TNF-α, IFN-y, erb-B2 und EGF.

Das erfindungsgemäße Verfahren läßt sich unabhängig vom Stadium eines Krebses anwenden. Es kann allein oder in Kombination mit anderen Krebsdiagnoseverfahren, wie bildgebenden oder auf herkömmlichen Tumormarkern basierenden Verfahren, eingesetzt werden. Das erfindungsgemäße Verfahren kann zur Prävention, beim Auftreten erster Warnzeichen für Krebs oder beispielsweise nach einer Therapie eines Krebses zur Rezidiv-Früherkennung eingesetzt werden. Es eignet sich zur Charakterisierung und Identifizierung sämtlicher Krebsformen, soweit entsprechende zirkulierende Krebszellen in den untersuchten Körperflüssigkeiten vorhanden sind. Hierzu gehören beispielsweise Abdominalkrebs, Analkrebs, Beckenkrebs, Gallengangkrebs, Gebärmutterkrebs, Gebärmutterschleimhautkrebs, Gehirnkrebs, Kopf- und Nackenkrebs, Lippenkrebs, Mundkrebs, Nierenkrebs, Parotiskrebs, Zungenkrebs, Leistenkrebs, Weichteilkrebs, Lymphome, Leukämien, multiple Leukämien, und bevorzugt Mammakarzinome, Sarkome, Ovarkarzinome, Lungenkarzinome, Pankreaskarzinome, Colonkarzinome, Rectumkarzinome, Prostatakarzinome, Leberkarzinome, Blasenkarzinome, Magenkarzinome, Schilddrüsenkarzinome, Cervixkarzinome, Endometriumkarzinome, Melanome, Non-Hodgkin-Lymphone und chronische myeloische Leukämien.

Insbesondere interessant ist der Einsatz des erfindungsgemäßen Verfahrens bei lymphknotenfreien Krebsformen, da in diesem Fall herkömmliche, auf der Untersuchung von Lymphknoten basierende Verfahren versagen. Werden zirkulierende Krebszellen bei NO-Tumoren (beispielsweise einem Mamma- oder Colonkarzinom) nachgewiesen, sind aufgrund der besonderen Konstellation gezielte Aussagen zur Therapiewahl möglich. So ist in diesen Fällen vorzugsweise eine adjuvant-kurative Therapie angezeigt, bei fortgeschrittenen Tumoren gegebenfalls mit einer nachgeschalteten zusätzlichen immunomodulatorischen Therapie.

Unabhängig davon, ob nur auf krebsspezifische oder zusätzlich auch auf krebsassoziierte Gene untersucht wird, wird erfindungsgemäß auf wenigstens zwei verschiedene Gene untersucht, so dass erfindungsgemäß ein Verfahren zur multiplen Charakterisierung disseminierter und mikrometastasierter Krebszellen bereitgestellt wird.

Eine erste Anwendung des erfindungsgemäßen Verfahrens richtet sich auf den Nachweis zirkulierender Krebszellen. Zu diesem Zweck wird vorzugsweise die Expression krebsspezifischer Gene gemessen. Besonders bevorzugt sind Multiparameter-Expressionsanalysen solcher Genen, die bei Nicht-Krebszellen der untersuchten Körperflüssigkeit abgeschaltet sind. Diese Analysen können bis zu etwa 40 Gene umfassen. In der Regel werden bis zu etwa 25 Gene, vorzugsweise etwa 2 bis 10 Gene und insbesondere etwa 3 bis 7 Gene untersucht. Vorzugsweise werden die entsprechenden mRNA's, insbesondere durch RT-PCR, analysiert.

Besonders effektive Kombinationen umfassen die Gene *C*EA und CK20, wobei die Analyse der entsprechenden mRNA's bevorzugt ist. Diese Kombinationen können gegebenfalls durch eine Untersuchung auf MUC1 vorteilhaft ergänzt werden, wobei man insbesondere die Relation zwischen der tumorspezifischen 336BP-Splicevariante und der natürlichen 309BP-Splicevariante analysiert. Derartige Untersuchungen sind besonders zum Nachweis zirkulierender Krebszellen vom Karzinomtyp brauchbar. Bevorzugt sind in diesem Zusammenhang gynäkologische Karzinome, wie Ovar-, Mamma- oder verschiedene Gebärmutterkarzinome, Colonkarzinome, Lungenkarzinome, Magenkarzinome, Schilddrüsenkarzinome, Blasenkarzinome Endometriumkarzinome und Prostatakarzinome. Die Untersuchung kann ohne vorherige Abtrennung der Krebszellen erfolgen. Bei Blutuntersuchungen verwendet man bevorzugt die MNC-Fraktion.

Weitere effektive Kombinationen umfassen das MAGE3- und Tyrosinase-Gen, wobei die Analyse der entsprechenden mRNA's bevorzugt ist. Diese Kombinationen können gegebenenfalls durch eine Untersuchung auf Muc18 vorteilhaft ergänzt werden. Derartige Untersuchungen sind insbesondere zum Nachweis zirkulierender Krebszellen vom Melanomtyp brauchbar.

Die vorstehend genannten Expressionsanalysen können durch weitere Nachweisverfahren krebsspezifischer Gene ergänzt werden. Zu diesem Zweck werden vorzugsweise Untersuchungen auf Onkogene und/oder mutierte Tumorsuppressor-Gene angestellt, wobei insbesondere auf die oben genannten erfindungsgemäß bevorzugten Gene dieser Art zurückgegriffen werden kann. Derartige Analysen, insbesondere zum Nachweis von Mutationen, Amplifikationen, LOH's, Translokationen oder Polymorphismen, werden vorteilhafterweise auf DNA-Ebene, beispielsweise durch DNA-Sequenzierung- oder Hybridisierungstechniken, durchgeführt und können bis zu etwa 40 Gene umfassen. In der Regel werden bis zu etwa 20 Gene, vorzugsweise etwa 2 bis 10 Gene und insbesondere etwa 3 bis 7 Gene untersucht.

Besonders effektive Kombinationen umfassen die Gene p53 und/oder erb-B2. Dabei wird p53 vorzugsweise anhand der entsprechenden cDNA auf Mutationen und/oder LOH und erb-B2 vorzugsweise auf DNA-Ebene auf Amplifikationen untersucht. Diese Kombinationen können gegebenfalls durch Untersuchungen auf c-myc und/oder K-ras, wobei c-myc bevorzugt auf DNA-Ebene auf Amplifikation und K-ras auf Mutationen untersucht wird, und/oder durch Unteruchungen auf RB, APC, DCC und/oder DPC4, vorzugsweise anhand von LOH's, vorteilhaft ergänzt werden.

Diese erste Anwendung kann dadurch ergänzt werden, daß man die zirkulierenden Krebszellen auf Gene untersucht, die Aussagen über deren Ursprung zulassen, d.h. eine Organlokalisation der Streuquelle erlauben. Dies kann auch in Form von Multiparameter-Expressionsanalysen geschehen, bei denen organotypische Morphogene gemessen werden. Solche Analysen können bis zu etwa 36 Gene umfassen. In der Regel werden bis zu etwa 14 Gene, vorzugsweise etwa 1 bis 8 und insbesondere 2 bis 5 Gene untersucht.

Besonders effektive Kombinationen umfassen die Maspin- und/oder PR-Gene insbesondere für den Nachweis von Mammakarzinomen, wobei vorzugsweise die entsprechenden mRNA's analysiert werden. Vorteilhaft ergänzt werden kann diese Kombination durch Untersuchungen auf β-hCG und/oder ÖR. Analoges gilt für den Nachweis von Ovar- und Cervix-Karzinomen, wobei in diesem Fall vorteilhaft durch eine Untersuchung auf SCCA ergänzt werden kann.

Weitere besonders effektive Kombinationen umfassen die PSM- und/oder PSA-Gene insbesondere für den Nachweis von Prostatakarzinomen, wobei vorzugsweise die entsprechenden mRNA's analysiert werden. Vorteilhaft ergänzt werden kann diese Kombination durch Untersuchungen auf hK2.

Weitere besonders effektive Kombinationen umfassen das Gastrin-Gen insbesondere für den Nachweis von Colonkarzinomen, wobei vorzugsweise die entsprechende mRNA analysiert wird. Auch eine Kombination aus GIP und/oder Motilin bietet eine effektive Nachweismöglichkeit von Colonkarzinomen.

Weitere besonders effektive Kombinationen umfassen SP-A und SP-C insbesondere für den Nachweis von Lungenkarzinomen, wobei vorzugsweise die entsprechende mRNA analysiert wird. Vorteilhaft ergänzt werden kann diese Kombination durch Untersuchungen auf βhCG.

Weitere besonders effektive Kombinationen umfassen EGF-R und βhCG insbesondere für den Nachweis von Blasenkarzinomen.

Eine weitere Anwendung des erfindungsgemäßen Verfahrens betrifft die Erstellung eines Risikoprofils nachgewiesener zirkulierender Krebszellen, aufgrund dessen eine Prognose gestellt werden kann. Zu diesem Zweck werden vorzugsweise die Metastasierungseigenschaften dieser Krebszellen bewertet.

Das Risikopotential des Tumors ist außerdem durch die Analyse von Mutationen und Amplifikationen und/oder verstärkter/verminderter Expression bestimmter Gene zu beschreiben, welche das Wachstumsverhalten der Krebszellen beeinflußen (z.B.: c-myc, c-erb-B2, c-fos, erb-B, mdm2, nm23, p16, p21).

Ein weiterer wichtiger Faktor zur Risikoeinschätzung ist die Empfindlichkeit des Tumors gegen immunologische Attacken des betroffenen Organismus. Viele Effektormechanismen bedingen die Apoptose der Zielzelle (Tumorzelle). Wenn ein Tumor diesen Abwehrmechanismen widersteht, ist das für diesen ein enormer Vorteil. Apoptose-relevante Gene können anzeigen, inwieweit ein Tumor gegen die Attacken der Abwehrzellen resistent oder empfindlich ist, oder eventuell sogar selbst Attacken gegen die Effektorzellen ausüben kann (z.B.: Perforin, Granzym, bax, bcl-2, fas, fas-L, GADD45, p53, TNF-R1, TNF-R2 ).

Von ebenso wichtiger Bedeutung ist die Quantifizierung der Tumorzellen im Blut. Es ist entscheidend, ob sich die Anzahl zirkulierenden Tumorzellen vor und nach operativem Eingriff oder Therapie unterscheiden. Eine Quantifizierung der Krebszellen anhand der oben angegebenen krebsspezischen Gene mit Hilfe von Longitudinalstandards erlaubt eine solche Aussage.

Zur Erstellung eines solchen Risikoprofils werden Multiparameter-Expressionsanalysen bevorzugt. Diese können bis zu etwa 50 Gene umfassen. In der Regel werden bis zu etwa 25 Gene, vorzugsweise etwa 2 bis 15 Gene und insbesondere etwa 4 bis 12 Gene untersucht.

Zur Risikoabschätzung insbesondere bevorzugt ist die Bewertung der Metastasierungseigenschaften; man stellt dabei vorzugsweise auf die Fähigkeit der Krebszellen zur Matrixdegradation und zur Steuerung der Angiogenese ab. In diesem Zusammenhang greift man insbesondere auf die oben genannten Angiogenesefaktoren und/oder Proteasen sowie deren Gegenspieler zurück.

Besonders effektive Kombinationen zur Charakterisierung der Metastasierungseigenschaften umfassen bFGF, bFGF-R, VEGF-R1 und/oder VEGF-R2 gegebenfalls zusammen mit VEGF, wobei bevorzugt die entsprechenden mRNA's untersucht werden. Diese Kombinationen können gegebenfalls durch Untersuchungen auf MMP's, insbesondere MMP2, und/oder TIMP's, insbesondere TIMP3, ergänzt werden, wobei man auch hier bevorzugt die entsprechenden mRNA's untersucht.

Effektive Kombinationen zur tumorbiologischen Untersuchung umfassen die FAS-L- und FAS-R-Gene, die vorzugsweise anhand der entsprechenden mRNA's untersucht werden. Vorteilhaft ergänzt werden kann diese Kombination durch Untersuchungen auf Cycline, insbesondere Cyclin B1, D1 und E, Ki67, bax und/oder bcl-2.

Ein besonderer Vorteil des erfindungsgemäßen Verfahrens liegt darin, daß sich individuelle Risikoprofile für einzelne Patienten erstellen lassen. Da das Verfahren insbesondere zur kontinuierlichen Anwendung geeignet, d.h. jederzeit wiederholbar ist, können anhand der Veränderung solcher Risikoprofile wertvolle Aussagen über die Entwicklung eines Krebses für einen individuellen Patienten getroffen werden. Ein weiterer Vorteil ist, daß somit nicht auf Statistiken zurückgegriffen werden muß, die im allgemeinen auf Erhebungen beruhen, bei denen Patienten mit sehr unterschiedlichen Voraussetzungen gemittelt werden.

Eine weitere Anwendung des erfindungsgemäßen Verfahrens betrifft die Therapie eines nachgewiesenen Krebses. So lassen sich Aussagen zur Therapiewahl, -kontrolle und -resistenz treffen.

Für die Therapiewahl sind Fragestellungen wichtig, welche die Therapieform oder die Medikamentenwahl betreffen. Hierzu gehören beispielsweise Entscheidungen, ob kurativ oder palliativ, adjuvant oder risikoadaptiert therapiert werden soll, sowie die Beurteilung der Wirksamkeit einer Anti-Krebstherapie. Beispielsweise lassen sich Anti-Krebsmittel (Cytostatika), die zum programmierten Zelltod (Apoptose) führen, auf ihre Wirksamkeit prüfen, indem man apoptoseassoziierte Gene untersucht. Insbesondere eignen sich zu diesem Zweck die Analyse verschiedener mRNA's oben beschriebener apoptoseassoziierter Gene. Diese Tests werden vorzugsweise an zirkulierenden Krebszellen durchgeführt, die in-vitro kultiviert werden. Dem Patienten müssen somit keine Cytostatika verabreicht werden.

Ein weiteres Anwendungsgebiet ist daher auch die Verwendung erfindungsgemäß charakterisierter, disseminierter und mikrometastasierter Krebszellen zum Testen von Wirkstoffen auf antineoplastische Wirkung.

Grundsätzlich können mit dem erfindungsgemäßen Verfahren alle Anti-Krebstherapien bewertet werden. Hierzu zählen beispielsweise Vaccine, Immunmodulation, molekulare Therapien, wie Gen-Replacement, Antisense-Nucleotide, Ribozyme, monoklonale Antikörper, MMP-Inhibitoren und attenuierte Viren, z.B. E1B-attenuierte Viren zur Cytolyse p53wt-defizienter Krebszellen.

Da das erfindungsgemäße Verfahren auf molekularbiologischen Untersuchungen beruht, eignet es sich in hervorragender Weise dazu, Information bezüglich der Therapiewahl zu liefern, die der molekularen Ausstattung der untersuchten Krebszellen angepaßt sind.

Es ist davon auszugehen, daß eine erfolgreiche Anti-Krebstherapie zur Abnahme und günstigstenfalls zum vollständigen Verschwinden zirkulierender Krebszellen und/oder zum Verlust von Risikofaktoren führt. Spricht eine Krebsform auf eine bestimmte Therapie nicht an, ist in der Regel davon auszugehen, daß die Zahl der zirkulierenden Krebszellen nicht ab- sondern ggf. zunimmt oder daß sich das patientenspezifische Risiko vergrößert. Es ist somit möglich, den Verlauf einer Krebserkrankung bzw. deren Therapie durch wiederholte Anwendung des erfindungsgemäßen Verfahrens zu bewerten. Durch einen zeitabhängigen Vergleich können somit die Wirksamkeit einer Therapie beurteilt und auf einfache Weise auch Resistenzen gegenüber bestimmten Therapieformen erkannt werden. Deren Auftreten kann darüber hinaus durch Untersuchungen zirkulierender Krebszellen auf Chemoresistenz-Gene nach Applikation von Therapeutika an den Patienten bestätigt werden.

Die Analyse verschiedener Zielgene bestimmter Therapeutika, wie EGP (Antikörper gegen epitheliales Antigen), c-erb-B2 (Anti-erb-B2-Antikörper-Mustard Komplex), MMP (Anti-Protease-Therapie), PR und ÖR (Anti-Hormon-Therapie), bFGF (Anti-bFGF-Therapie) oder Topoisomerase II (Doxorubicin u.a.) können Aussagen über die spezifische Wirkung der Substanzen erlauben, indem die "Targetparameter" direkt quantifiziert werden. Der Erfolg einer cytostatischen Therapie mit mikrotubulistabilisierenden Taxanen (z.B. Taxol) kann durch den Nachweis der RNA-Expressiön der monomeren Targetmoleküle (α-und β-Tubuline), deren Assembly unter Taxol verhindert wird, vorhergesagt werden. Eine Resistenz von Krebszellen gegen Cytostatika (z.B. Cispaltin) kann durch den Verlust der Expression von DNA-Reparaturgenen (z.B. hMLH1) nachgewiesen werden. Weiterhin kann durch die Erzeugung von in-vitro Systemen an patienteneigenen Tumorzellen direkt die Wirkung verschiedenster Therapeutika vorgetestet werden, um auf diese Weise die bestmögliche Behandlungsform zu ermitteln.

Besonders effektive Kombinationen zur Beurteilung einer möglicherweise bestehenden Chemoresistenz umfassen die Gene MDR1, MRP, Topoisomerase II und Glutathion-S-Transferase-pi, wobei man beispielsweise die entsprechenden mRNA's mißt. Ergänzend können auch β-Tubulin-Mutationen und MDR1-Amplifikation untersucht werden. Bezüglich der MDR1-Untersuchung greift man auch häufig auf die Analyse der MDR1-Pumpe-gp170 und/oder den MDR1-Efflux-Doxorubicin-Test zurück.

Ein besonderer Vorteil des erfindungsgemäßen Verfahrens besteht darin, daß therapierefraktäre Zellen (minimal residual disease; MRD) charakterisiert und identifiziert werden können und in Anlehnung daran ein schon ausgeführter Therapieansatz risikoadaptiert erweitert werden kann, um die residuellen Krebszellen vollständig zu eliminieren.

Obwohl das erfindungsgemäße Verfahren in erster Linie im Hinblick auf die Charakterisierung humaner Gene beschrieben worden ist, soll es nicht darauf beschränkt sein. Vielmehr erschließen sich dem Fachmann eine Reihe weiterer Anwendungen, wie die in Tiermodellen zur Beantwortung von Fragestellungen, die den obigen entsprechen oder zumindest ähnlich sind.

Mittel zur Durchführung des erfindungsgemäßen Verfahrens sollten möglichst einfach handhabbar und im Wesentlichen gebrauchsfertig sein. Vorteilhaft verwendet man Mittel zur Durchführung des erfindungsgemäßen Verfahrens in Kit-Form, beispielsweise als Test- und/oder Diagnosekit. Ein solcher Kit beinhaltet wenigstens ein Kompartiment, beispielsweise ein Vial oder Teströhrchen, in dem die Mittel zur erfindungsgemäßen Untersuchung auf obige Gene möglichst in aliquotierten Mengen enthalten sind. Üblicherweise umfaßt der Kit mehrere Kompartimente, wobei ein Kompartiment der Untersuchung auf ein bestimmtes Gen zugeordnet sein kann, aber auch Mittel umfassen kann, die zur Untersuchung mehrerer Gene verwendet werden können. Unter Umständen können auch mehrere Kompartimente der Untersuchung auf ein bestimmtes Gen zugeordnet sein. Gegebenenfalls umfasst der Kit auch ein weiteres Kompartiment zur Aufnahme der Körperflüssigkeitsprobe. Der Kontakt der Körperflüssigkeitsprobe mit den Mitteln zur Durchführung des erfindungsgemäßen Verfahrens kann gegegebenfalls in einem weiteren Kompartiment stattfinden. Die Wahl der Mittel richtet sich nach den untersuchten Genen und der gewählten Methode.

Erfindungsgemäße Diagnose- und/oder Testkits können Mittel zur Aufbereitung der Körperflüssigkeitsprobe, z.B. Mittel zur Anreicherung von Zellen aus Körperflüssigkeiten, wie Dichtegradienten und/oder Filter, Mittel zur Isolation und Reinigung von DNA und/oder RNA aus Zellen, insbesondere auf Guanidinisothiocynat basierende Systeme, Spin-Säulen mit geeigneten Festphasen und/oder Oligo-dT-Systeme; Mittel zur Durchführung der Reversen Transkription (RT), z.B. Reverse Transkriptase, RT-Puffer, RNase-Inhibitor, geeignete Primer und/oder dNTPs; Mittel zur Durchführung der PCR, z.B. thermostabile Poylmerase, PCR-Puffer, MgCl₂ und/oder dNTPs; Mittel zur Durchführung restriktionsenzymatischer Verdauungen (RV), z.B. Restriktionsenzym und RV-Puffer; und/oder Mittel zur Analyse der mittels RT, PCR, und/oder RV erhaltenen Produkte, z.B. Gele bzw. Mittel für die Zubereitung zweckmäßiger Gele, ELISAs u.ä., enthalten.

Andererseits können viele der zur Durchführung obiger Methoden erforderlichen Mittel, häufig sogar in Kit-Form, auch im Handel bezogen werden, so daß es zur Durchführung des erfindungsgemäßen Verfahrens lediglich ergänzender Mittel bedarf. Derartige erfindungsgemäße Ergänzungskits stellen vorzugsweise zweckmäßige Primer, Sonden und/oder Negativ/Positivkontrollen sowie gegebenfalls weitere Hilfsmittel zur Verfügung.

Bevorzugt werden erfindungsgemäße Kits bzw. Ergänzungskits, welche die Untersuchung auf die oben beschriebenen als effektiv herausgestellten Genkombinationen ermöglichen. Derartige Kits werden mit der Zielsetzung verwendet, Krebs im allgeimenen nachzuweisen, Primärtumore zu lokalisieren, eine Risiko- und Prognoseabschätzung vorzunehmen oder Aussagen im Hinblick auf eine Therapie zu treffen. Für eine umfassende Beurteilung eines Krebses können derartige Kits in einer Art Baukastensystem kombiniert werden.

Andererseits können erfindungsgemäße Kits auch Mittel enthalten, die Untersuchungen auf Gene zur krebsspezifischen und krebsassoziierten Charakterisierung einer bestimmten Krebsart ermöglichen. Beispielsweise kann ein Melanomspezifischer Kit wenigstens Mittel zur Untersuchung auf Tyrosinase und bFGF, ein Colon-spezifischer Kit wenigstens Mittel zur Untersuchung auf CK20, bFGF und MMP2 und ein Mamma-spezifischer Kit wenigstens Mittel zur Untersuchung auf CK19, bFGF, MMP2 und PR enthalten.

Die nachfolgenden Beispiele sollen die Erfindung näher erläutern, ohne sie zu beschränken.

### Referenzbeispiel 1:

### Isolierung mononukleärer Zellen (MNC) aus Blut

Man befüllt ein Zentrifugenröhrchen (50 ml) mit 15 ml Dichtegradientenmedium (DGM) 1.077 (RT) und überschichtet vorsichtig mit 30 ml Blut/PBS (Heparinisiertes oder EDTA-Blut). Nach 30-minütiger Zentrifugation (800g, RT) werden alle Interphase-Zellen (MNC) mit einer Pasteurpipette in ein neues mit 6 ml PBS/Zus (PBS + BSA 0.2%, Natriumazid 0.02%, EDTA 1 mM) befülltes Zentrifugenröhrchen (15 ml) überführt. Ab hier werden alle Schritte unter Kühlung (4°C) durchgeführt. Es wird zentrifugiert (10 Min. 600g, 4°C), die Zellen werden in 10 ml PBS/Zus aufgenommen und die Zellzahl durch Trypan-Blau-Färbung (95 µl TB-Lösung + 5 µl Zellösung) bestimmt (Zellzahl = Zellzahl aus 16 Kleinquadraten (Neubauer) x 2x10⁶). Es wird zentrifugiert (10 Min. 400g, 4°C), die Zellen werden in eisgekühltem PBS/Zus aufgenommen und in ein Eppendorf-Reaktionsgefäß (1,5 ml) überführt (bei einer Zellzahl bis 2.5 E7 in 0.5 ml; bei 2.5 E7 bis 5E7 in 1 ml). Dann wird zur RNA/DNA-Isolation Fraktion A (1/4 Vol. bei 20 ml, 1/6 Vol. bei 30 ml) entnommen, die darin enthaltenene Zellen werden zentrifugiert (400g, 3 min), das resultierende Pellet wird in 600 µl RLT-Me-Puffer (aus RNEasy Blood Kit Quiagen) resuspendiert und bei -80°C gelagert. Schließlich wird 10%-iges FC-Blocking-Reagens zugegeben.

### Referenzbeispiel 2:

### Isolierung epithelialer Tumorzellen aus Vollblut

Anti-Epithelial-Beads (40 µl/0.5 ml) werden zweimal mit 800 µl PBS/Zus in einer Magnetleiste gewaschen. 40 µl/0.5 ml gewaschene Beads werden in ein Eppendorf-Reaktionsgefäß gegeben, man läßt das Reaktionsgefäß in einem Rotor 25 min bei 4°C rotieren, und man stellt es dann in einen Reaktionsgefäß-Ständer, und die Suspension vom Reaktionsgefäß-Deckel wird in das Reaktionsgefäß gegeben. Das Reaktionsgefäß wird 1 min in MPC gegeben, die Zellsuspension wird verworfen, die Magnetleiste wird abgenommen (oder das Gefäß herausgenommen), es werden 800 µl PBS/Zus hinzugeben und vorsichtig resuspendiert. Die letzte Schrittfolge wiederholt man 6-mal, wobei zuletzt in PBS/1mM EDTA (ohne BSA) resuspendiert wird.
Das Reaktionsgefäß wird 1 min in MPC gegeben und der Überstand vollständig entfernt. Die resultierenden Beads mit anheftenden Zellen bilden die Fraktion C. Diese wird für die RNA/DNA-Isolation in 200 µl Trizol resuspendiert und bei -80°C gelagert. Als Reinheitmarker der Fraktion C kann man Perforin-mRNA messen.
Die Auswertung erfolgt über die Bestimmung tumorassoziierter und tumorspezifischer RNA sowie über die Bestimmung der RNA des epithelialen Glycoproteins mittels Quantifizierung der GAPDH-RNA.

### Referenzbeispiel 3:

### DNA/RNA-Isolierung

Die DNA/RNA-Isolierung wird in an sich bekannter Weise durchgeführt.

### Referenzbeispiel 4:

### CK20- und CEA-mRNA-Analyse mittels RT-PCR

Zum Nachweis von Epithelzellen im Blut wird der Gehalt an CK20-mRNA molekularbiologisch mit einer Sensitivität von mehr als 1 Zelle auf 10⁶ Leukozyten organselektiv (Ovar, Colon > Mamma) bestimmt. Außerdem wird der CEA-mRNA-Gehalt unter-sucht. Dieser Nachweis von Zellen, die das onkogene Adhaerin CEA bilden, wurde mit einer Sensitivität von einer Krebszelle auf 10⁶ Leukozyten geführt.

### Versuchsablauf

### Reverse Transkription (RT):

Folgende Reagenzien werden zusammengegeben (RT-Mix):
2, 35 µl H₂O
4 µl 5x First-Strand-Puffer
2 µl 0,1 M DTT
0,15 µl RNA-Guard (38950 U/ml)
0,5 µl Random-Primer (500 µg/ml)
0,5 µl dNTP-Mix (je 20 mM)
0,5 µl M-MLV (200 U/µl)

10 µl aus mononukleären Zellen (5 ml Blut) isolierte RNA (ca. 1 µg) werden 1 min bei 70°C denaturiert, sofort auf Eis 3 min abkühlt, mit 10 µl RT-Mix luftblasenfrei vermischt, 60 min bei 37°C inkubiert, 3 min bei 95°C inkubiert, sofort auf Eis 3 min abkühlt und entweder direct der PCR zugeführt oder bei -20 °C eingefroren.

### PCR:

Pro PCR-Ansatz werden folgende Reagenzien zusammengegeben (µl):

| | CK20 | CEA |
|---|---|---|
| TaqMan-Puffer (10x) | 5,0 | 5,0 |
| MgCl₂ (25 mM) | 8,0 | 8,0 |
| dNTP (je 0,75 µl; 2,5 mM) | 3,0 | 3,0 |
| Primer A (20 pmol/µl) | 0,75 | 0,75 |
| Primer B (20 pmol/µl) | 0,75 | 0,75 |
| Sonde TaqMan (20 pmol/µl) | 0,5 | 0,5 |
| Amplitaq-Gold (PE 5U/µl) | 0,5 | 0,5 |
| H₂O | 28,5 | 28,5 |
| cDNA aus RT | 3,0 | 3,0 |

Die PCR wird auf dem ABI 7700 Seqence Detector (TaqMan) durchgeführt. Verwendet wird eine zweistufige PCR-Methode. Folgendes Temperaturprofil wird benutzt:

| | |
|---|---|
| für CK20: | |
| 95° C | 12 min (Hot-Start-Aktivierung) |
| 95° C | 30 sec |
| 57° C* | 60 sec 45x |
| 20° C | ∞ |
| für CEA: | |
| 95° C | 12 min (Hot-Start-Aktivierung) |
| 95° C | 30 sec |
| 58° C* | 60 sec 45x |
| 20° C | ∞ |

### Kontrollen:

- Pos. (CK20):: NCI-H508-Adenokarzinom-Zelllinie
- Neg. (CK20):: MES-SA/Dx5-Uterussarkom-Zelllinie; Lymphozyten eines Normalspenders
- Pos. (CEA):: NCI-H508-Adenokarzinom-Zelllinie; MCF7-Mammakarzinom-Zelllinie
- Neg. (CEA):: Lymphozyten eines Normalspenders

**Primer:**

| | Primer A | Primer B | Taqman-Sonde |
|---|---|---|---|
| CK20 | CK20-sense | CK20-antisense | CK20-Sonde |
| CEA | CEA-sense | CEA-antisense | CEA-Sonde |

### Analyse der PCR-Produkte:

Die Ausbeute der ablaufenden PCR-Reaktion wird für jede Cyclus-Runde im Seqence Detector online gemessen. Die dabei aufgezeichnete Kurve des Reaktionsverlaufs dient als Grundlage für die Mengenbestimmung der zu analysierenden CDNA als Äquivalent der mRNA. Basis ist die Bestimmung des Übergangs der PCR-Reaktion in die exponentielle Phase.

### Referenzbeispiel 5:

### MUC1-mRNA-Analyse mittels RT-PCR

Es wird eine Bestimmung der MUC1-mRNA vorgenommen, um die karzinomspezifische Mucin-Transkription zu beurteilen.

### Versuchsablauf

### Reverse Transkription (RT):

Folgende Reagenzien werden zusammengegeben (RT-Mix):
4 µl 25 mM MgCl₂
2 µl PCR-Puffer II (10x)
2 µl 10 mM dCTP
2 µl 10 mM dGTP
2 µl 10 mM dATP
2 µl 10 mM dTTP
1 µl RNAse-Inhibitor (2000 U)
1 µl M-MLV (5000 U)
1 µl Random Hexamere (5 nmol)

5 µl aus mononukleären Zellen (5 ml Blut) isolierte RNA (ca. 1 µg) werden zu dem RT-Mix gegeben, 10 min bei Raumtemperatur, 15 min bei 42° C, 5 min bei 99° C und 5 min bei 5° C inkubiert.

Pro PCR-Ansatz werden folgende Reagenzien zusammengegeben (µl) :

| | MUC1 |
|---|---|
| PCR-Puffer II (10x) | 8,0 |
| MgCl₂ (25 mM) | 4,0 |
| Primer A (20 pmol/µl) | 2,0 |
| Primer B (20 pmol/µl) | 2,0 |
| Amplitaq-Gold (PE 5U/µl) | 0,75 |
| H₂O | 63,5 |
| cDNA aus RT | 22,0 |

Die PCR wird auf dem Thermocycler Perkin Elmer 9600 oder 2400 durchgeführt. Folgendes Temperaturprofil wird benutzt:

| | | |
|---|---|---|
| 95°C | 10 min | |
| 95°C | 15 sek | |
| 60°C | 30 sek | 35 Zyklen |
| 72°C | 7 min | |

Der PCR-Ansatz wird auf 4°C gekühlt.

### Kontrollen:

- Pos.:: MES-SA/Dx 5 (Uterussarkom-Zellinie); exprimiert nur 336 bp Splicevariante
- Neg.:: Lymphozyten eines Normalspenders; exprimieren nur 309 bp Splicevariante

### Primer:

| | Primer A | Primer B |
|---|---|---|
| MUC1 | MUC S1-sense | MUC S2-antisense |
| | (5'-6-FAM) | |

### Analyse der PCR-Produkte:

12 µl Formamid, 0,5 µl Genescan Tamra 500 (PE) und 1 µl PCR-Ansatz werden zusammengegeben, 3 min bei 95°C denaturiert, auf Eis inkubiert und im ABI Genescan 310 analysiert (Anodenpuffer: 2% Polymer, 1x Genetic Analyzer-Puffer; Kathodenpuffer: 3% Polymer, 40% Harnstoff, 1x Genetic Analyzer-Puffer; Injektionszeit: 10 s; Injektiosspannung: 7kV; Laufspannung: 13 kV; Lauftemperatur: 30°C; Laufzeit: 18 min; Modul: Short Denatured C).
Zur Auswertung werden die Peakflächen der PCR-Produkte (336 bp und 309 bp) ermittelt. Es wird der Quotient zwischen der Peakfläche der 336 bp-Splicevariante und der 309 bp-Splicevariante gebildet (0,2 ist normal, bis 0,7 enspricht einer schwachen Expression und > 0,7 einer starken Expression).

### Referenzbeispiel 6:

### GST-pi-, FAS-R-, FAS-L-, MMP-2-mRNA-Analyse mittels RT-PCR

### Versuchsablauf:

Die Reverse Transkription (RT) wird gemäß Referenzbeispiel 4 durchgeführt.

Pro PCR-Ansatz werden folgende Reagenzien zusammengegeben (µl):

| | GST-pi | FAS-R | FAS-L | MMP-2 |
|---|---|---|---|---|
| PCR-Puffer II (10x) | 5,0 | 5,0 | 5,0 | 5,0 |
| ROX TIB-MOLBIOL (100 µM) | 0,75 | 0,5 | 0,5 | 0,5 |
| MgCl₂ (25 mM) | 6,0 | 8,0 | 8,0 | 8,0 |
| dNTP (je 0,75 µl) 2,5 mM | 3,0 | 3,0 | 3,0 | 3,0 |
| Primer A (20 pmol/µl) | 1,0 | 1,0 | 0,75 | 0,75 |
| Primer B (20 pmol/µl) | 1,0 | 1,0 | 0,75 | 0,75 |
| Sonde TaqMan (20 pmol/µl) | 0,5 | 0,3 | 0,5 | 0,5 |
| Amplitaq-Gold (PE 5U/µl) | 0,5 | 0,5 | 0,5 | 0,5 |
| H₂O | 29,25 | 27,7 | 28,0 | 28,0 |
| cDNA aus RT | 3,0 | 3,0 | 3,0 | 3,0 |

Die PCR wird auf dem ABI 7700 Seqence Detector (TaqMan) durchgeführt. Verwendet wird eine zweistufige PCR-Methode. Folgendes Temperaturprofil wird benutzt:

| | |
|---|---|
| für GST-pi: | |
| 95° C | 10 min (Hot-Start-Aktivierung) |
| 95° C | 30 sec |
| 57° C | 60 sec 40 x |
| 20° C | ∞ |
| für FAS-R | und FAS-L: |
| 95° C | 12 min (Hot-Start-Aktivierung) |
| 95° C | 30 sec |
| 55° C | 30 sec 45 x |
| 20° C | ∞ |
| für MMP-2: | |
| 95° C | 10 min (Hot-Start-Aktivierung) |
| 95° C | 30 sec |
| 58° C | 60 sec 45 x |
| 20° C | ∞ |
| Cyclenanzahl: | (45 für FAS; MMP-2) |

### Kontrollen:

- Pos. (GST-pi):: MES
- MNC (GST-pi):: Mittels des CT-Wertes der MNC-Kontrolle wird der Grenzwert der normalen Expression festgelegt.
- Pos. (FAS-R):: MNCs (Normalspender); ES-2; MNC-cDNA-Verdünnungsreihe
- Neg. (FAS-R):: A. dest.
- Pos. (FAS-L):: MNCs (Normalspender); ES-2; MNC-cDNA-Verdünnungsreihe
- Neg. (FAS-L):: MCF-7 (Mamma-Karzinom); Daudi (Burkitt-Lymphom)
- Pos. (MMP-2):: COLO-320
- Neg. (MMP-2):: SW403; Lymphozyten eines Normalspenders

### Primer:

| | Primer A | Primer B | TaqMan-Sonde |
|---|---|---|---|
| GST-pi | GST-pi-sense | GST-pi-antisense | GST-pi-Sonde |
| FAS-R | FAS-R-sense | FAS-R-antisense | FAS-R-Sonde |
| FAS-L | FAS-L-sense | FAS-L-antisense | FAS-L-Sonde |
| MMP-2 | MMP-2-sense | MMP-2-anitsense | MMP-2-Sonde |

Primer- und Sondesequenzen für FAS wurden entworfen nach GenBank Accession # M67454; Primer- und Sondesequenzen für FAS-L wurden entworfen nach GenBank Accession # U08137.

### Analyse der PCR-Produkte:

Analog Referenzbeispiel 4.

### Referenzbeispiel 7:

### bFGF-R-, bFGF-, VEGF-R2-mRNA-Analyse mittels RT-PCR

### Versuchsablauf:

Die Reverse Transkription (RT) wird gemäß Referenzbeispiel 1 durchgeführt.

Pro PCR-Ansatz werden folgende Reagenzien zusammengegeben (µl):

| | bFGF-R | bFGF | VEGF-R2 |
|---|---|---|---|
| PCR-Puffer (10x) | 5,0 | 5,0 | 5,0 |
| dNTP (20 mM) | 0,5 | 0,5 | 0,5 |
| Primer A (20 pmol/µl) | 1 | 1 | 1 |
| Primer B (20 pmol/µl) | 1 | 1 | 1 |
| Taq-Polymerase (PE 5U/µl) | 0,25 | 0,25 | 0,25 |
| H₂O | 31,55 | 31,55 | 31,55 |
| cDNA aus RT | 5,0 | 5,0 | 5,0 |

Die PCR wird auf dem Thermocycler Perkin Elmer 9600 oder 2400 durchgeführt. Folgendes Temperaturprofil wird benutzt:

| | |
|---|---|
| 95°C | 5 min |
| 95°C | 60 sek |
| 53°C | 60 sek |
| 72°C | 60 sek 45 x |
| 72°C | 10 min |

Der PCR-Ansatz wird auf 4°C gekühlt.

### Kontrollen:

- Pos. (bFGF-R):: ES-2 (Ovarkarinom); NB-4 (ApML); MCF-7 (Mammakarzinom); 697 (cALL); Colo 829 (Melanom)
- Neg. (bFGF-R):: Lymphozyten (Normalspender); NCI-H508 (Adenokarzinom); K562 (CML)
- Pos. (bFGF):: ES-2 (Ovarkarzinom); K562 (CML); MES-SA/Dx5 (Uterussarkom); Colo 829 (Melanom)
- Neg. (bFGF):: Lymphozyten (Normalspender); 697 (cALL); NCI-H508 (Adenokarzinom)
- Pos. (VEGF-R2):: ES-2 (Ovarkarzinom); Colo 829 (Melanom); EFM192A (Mammakarzinom)
- Neg. (VEGF-R2):: Lymphozyten (Normalspender); NCI-H508 (Adenokarzinom)

### Primer:

| | Primer A | Primer B |
|---|---|---|
| bFGF-R | bFGF-R-sense | bFGF-R-antisense |
| bFGF-L | bFGF-L-sense | bFGF-L-antisense |
| VEGF-R2 | VEGF-R2-sense | VEGF-R2-antisense |

### Analyse der PCR-Produkte

a) 20-30 µl des PCR-Ansatzes werden auf einem 2%-igen Agarose-Gel (1xTBE) aufgetrennt.
b) 6-10 µl des PCR-Produktes werden mit (+) oder ohne (-) Restriktionsenzym 1 Stunde bei 37°C inkubiert. Nach Auftrennung in 2%-igem Agarosegel sind zwei kleinere Fragmente zu erkennen.

| Restriktion | + | - |
|---|---|---|
| Reagenzien | 6 (10) µl PCR-Produkt 0,5 µl R-Enzym 1 (1,5) µl Puffer M 2,5 (3) µl A. dest. | 6 (10) µl PCR-Produkt - 1 (1,5) µl Puffer M 3 (3,5) µl A. dest. |

### Referenzbeispiel 8:

### Tyrosinase-mRNA-Analyse mittels RT-PCR

### Versuchsablauf:

Die Reverse Transkription (RT) wird gemäß Referenzbeispiel 1 durchgeführt.

Pro PCR-Ansatz werden folgende Reagenzien zusammengegeben (µl):

| | 1. Runde | 2. Runde |
|---|---|---|
| PCR-Puffer (10x) | 5,0 | 5,0 |
| MgCl₂ (0,1 M) | 0,8 | 0,8 |
| Triton X-100 (1%) | 5,0 | 5,0 |
| dNTP (20 mM) | 0,5 | 0,5 |
| Primer A (20 pmol/µl) | 7,5 | - |
| Primer B (20 pmol/µl) | 7,5 | - |
| Primer C (20 pmol/µl) | - | 7,5 |
| Primer D (20 pmol/µl) | - | 7,5 |
| Taq-Polymerase (5U/µl) + Taq-Antikörper 1:1 | 1,0 | 1,0 |
| H₂O | 20,7 | 20,7 |
| cDNA aus RT | 2,0 | - |
| PCR-Produkt aus 1. Runde | - | 2,0* |

| | | |
|---|---|---|
| * Positivkontrolle 100-fach verdünnen | | |

Die PCR wird auf dem Thermocycler Perkin Elmer 9600 oder 2400 durchgeführt. Folgendes Temperaturprofil wird benutzt:

| | | |
|---|---|---|
| 95°C | 10 min | |
| 95°C | 60 sek | |
| 60°C | 30 sek | |
| 72°C | 60 sek | 30 x |
| 72°C | 10 min | |

Der PCR-Ansatz wird auf 4°C gekühlt.

### Kontrollen:

- Pos. (Tyrosinase):: COLO-829 cDNA (Humanes Melanom); 697 cDNA (Pre-B-Zell-Leukämie)
- Neg. (Tyrosinase):: Prämix ohne Proben-DNA (Obligat); MES (Uterussarkom); LNCAP (Prostatakarzinom)

### Primer:

| | Primer A | Primer B | Primer C | Primer D |
|---|---|---|---|---|
| Tyrosinase | HTYR-1 | HTYR-2 | HTYR-3 | HTYR-4 |

### Analyse der PCR-Produkte:

10 µl PCR-Produkt und 1,1 µl Proben-Puffer werden zusammen mit 10 µl 100 bp-Leiter einer Agarose-Gelelektrophorese bei einer Laufspannung von 150 Volt und einer Laufzeit von 20 min unterzogen. Die Auswertung erfolgt unter einer UV Lampe bei 254 nm oder 312 nm. Das PCR-Produkt nach der 1. Runde besitzt eine Größe von 284 bp. Das PCR Produkt nach der 2 Runde besitzt eine Größe von 207 bp. Als Längenstandard wird die 100 bp-Leiter verwendet.

### Referenzbeispiel 9:

### erb-B2-, c-myc- und mdr1-Amplifikationsanalyse

Es werden das erb-B2-, c-myc und mdrl-Gen bzw. das β-Globin-Gen unter Verwendung von Fluorescein-markierten Oligonukleotidprimern koamplifiziert. Die Auftrennung der Amplifikate erfolgt mittels Kapillarelektrophorese.

### Versuchsablauf:

Pro PCR-Ansatz werden folgende Reagenzien zusammengegeben (µl):

| | erb-B2 | c-myc | mdr1 |
|---|---|---|---|
| PCR-Puffer (10x, PE) | 5,0 | 5,0 | 5,0 |
| MgCl₂ (25 mM, PE) | 4,0 | 4,0 | 0,5 |
| dNTP (20 mM) | 0,25 | 0,25 | 0,25 |
| (NH₄)₂SO₄ (100 mM) | 7,5 | 7,5 | 7,5 |
| Primer A (20 pmol/µl) | 1,5 | 2,0 | 5,0 |
| Primer B (20 pmol/µl) | 1,5 | 2,0 | 5,0 |
| Primer c (20 pmol/µl) | 0,5 | 0,2 | 0,5 |
| Primer d (20 pmol/µl) | 0,5 | 0,2 | 0,5 |
| Amplitaq-Gold (PE, 5U/µl) | 0,4 | 0,4 | 0,25 |
| H₂O (bidest) | 25,85 | 25,45 | 23,5 |
| DNA | 3,0 | 3,0 | 2,0 |

Die PCR wird auf einem Thermocycler Perkin Elmer 2400, 9600 oder 9700 durchgeführt. Folgendes Temperaturprofil wird benutzt:
für erb-B2 und c-myc:

| | | |
|---|---|---|
| 95°C | 10 min | |
| 95°C | 1 min | |
| 60°C | 1 min | |
| 72°C | 1 min | 32 x |
| 72°C | 3 min | |

für mdrl:

| | | |
|---|---|---|
| 94°C | 5 min | |
| 95°C | 1 min | |
| 57°C | 1 min | |
| 72°C | 1 min | 35 x |
| 72°C | 5 min | |

Der PCR-Ansatz wird auf 4°C gekühlt.

### Kontrollen:

Pos. (erb-B2): DNA-A
Norm. (erb-B2): humane DNA
Pos. (c-myc): DNA-B; H82-DNA
Norm. (c-myc): humane DNA
Pos. (mdr1): CCRF-DNA
Norm. (mdr1): humane DNA

### Primer:

| | Primer A | Primer B | Primer C | Primer D |
|---|---|---|---|---|
| erb-B2 | Hex-neu-3 | neu-5 | PCO-3F | PCO-4 |
| c-myc | Hex-myc-1 | myc-2 | PCO-3F | PCO-4 |
| mdrl | Hex-mdr1-5F | mdr1-5B | PCO-3F | PCO-4 |

### Analyse der PCR-Produkte:

Die PCR-Produkte werden auf ein 2%-iges Agarosegel aufgetragen. Nach der Kapillarelektrophorese (Genetic-Analyzer ABI 310) wird für jede Patientenprobe und die Kontrollen der Quotient aus erb-B2- c-myc- bzw. mdrl-Flächenintegral und β-Globin-Flächenintegral gebildet. Eine erb-B2- c-myc- bzw. mdrl-Amplifikation in einer Patientenprobe liegt dann vor, wenn der Quotient "signifikant" größer ist als der der Normalkontrolle, bzw. von gleichzeitig mitgemessenen Proben.

### Referenzbeispiel 10:

### DCC-, APC-, RB, p53-, Mikrosatelliten (DxSy)-LOH-Analysen

### Versuchablauf:

Pro PCR-Ansatz werden folgende Reagenzien zusammengegeben (µl):

| | DCC | APC | RB | p53 | DxSy |
|---|---|---|---|---|---|
| PCR-Puffer (10x) | 5,0 | 5,0 | 5,0 | 5,0 | 3,0 |
| dNTP-Mix (20 mM; 10 mM für DxSy) | 0,5 | 0,5 | 0,5 | 0,5 | 4,8 |
| MgCl₂ (25 mM) | - | - | - | 3,0 | 3,0 |
| Primer A (20 pmol/µl) | 0,5 | 0,5 | 0,5 | 0,5 | 0,6 |
| Primer B (20 pmol/µl) | 0,5 | 0,5 | 0,5 | 0,5 | 0,6 |
| Taq-Polymerase (5U/µl) + Taq-Antikörper 1:1 | 0,5 | 0,5 | 0,5 | 0,5 ** | 0,3 ** |
| H₂O | 40,0 | 40,0 | 40,0 | 37, 0 | 14,7 |
| DNA* | 3,0 | 3,0 | 3,0 | 3,0 | 3,0 |

| | | | | | |
|---|---|---|---|---|---|
| * 3µl DNA (Fraktion C; Tumorzellfraktion; Abstrich) 1 µl DNA (Fraktion A) + 2 µl H₂O ** Taq-Polymerase Amplitaq Gold | | | | | |

Die PCR wird auf dem Thermocycler Perkin Elmer 9600 oder 2400 durchgeführt. Folgendes Temperaturprofil wird benutzt:
Für DCC, APC und RB:

| | | |
|---|---|---|
| 95°C | 5 min | |
| 94°C | 30 sek | |
| 53°C | 30 sek | |
| 72°C | 30 sek | 30 x |
| 72°C | 5 min | |
| Für p53: | | |
| 94°C | 10 min | |
| 94°C | 30 sek | |
| 60°C | 30 sek | |
| 72°C | 30 sek | 40 x |
| 72°C | 5 min | |

Für DxSy:

| | | |
|---|---|---|
| 95°C | 10 min | |
| 94°C | 1 min | |
| 60°C | 30 sek | (D17S849 / D19S960) |
| 53°C | 30 sek | (D16S265 / D11S528) |
| 57°C | 30 sek | (D17S926 / D17S960) |
| 72°C | 30 sek | 40 x |
| 72°C | 10 min | |

Der PCR-Ansatz wird auf 4°C gekühlt.

### Kontrollen:

Neg.: Ansatz ohne DNA

### Primer:

| | Primer A | Primer B |
|---|---|---|
| DCC-LOH | Hex-DCC LOH-1 | DCC LOH-2 |
| RB-LOH | FAM-RB LOH-1 | RB LOH-2 |
| APC-LOH | FAM-APC LOH-1 | APC LOH-2 |
| p53-LOH | FAM-p53 LOH-A | p53 LOH-B |
| D17S926-LOH | FAM-D17S926 a | D17S926 s |
| D17S695-LOH | FAM-D17S695 a | D17S695 s |
| D17S849-LOH | FAM-D17S849 a | D17S849 s |
| D17S960-LOH | FAM-D17S960 a | D17S960 s |
| D16S265-LOH | FAM-D16S265 a | D16S265 s |
| D11S528-LOH | FAM-D11S528 a | D11S528 s |

### Analyse der PCR-Produkte:

### 1. Funktionsnachweis der PCR im Agarosegel

9 µl (15 µl für DxSy) PCR-Produkt und 1 µl (2 µl) 10x TBE-Probenpuffer mit Bromphenolblau werden auf ein 2%iges Agarosegel (10 µl Ethidiumbromid-Stammlösung 10 mg/ml auf 100 ml Gel) in 1 x TBE-Puffer aufgetragen. Die Elektrophorese wird 160 bis 170 V bei konstanter Spannung durchgeführt.

### 2. Nachweis des Allelverlustes mittels ABI 310

12 µl Formamid, 1 µl Probe und 0,5 µl Genescan Tamra 500 (PE) werden 2 min bei 95°C denaturiert und direkt auf Eis gestellt. Anschließend wird eine Fragmentanalyse auf dem ABI 310 (Analysepuffer; Anode und Kathode: 1x Fragmentanalysenpuffer (PE) mit EDTA; Polymer: POP-4; Injektionszeit: 5 sec; Injektionsspannung: 15 kV; Spannung während des Laufes: 15 kV; Temperatur während des Laufes: 60°C; Laufzeit: 24 min; Matrix: GS POP4C) durchgeführt.

Die Analyse wird sowohl im Gesamtblut, welche das Allelverhältnis in den Normalzellen widerspiegelt, als auch in der Fraktion C, die stellvertretend den Zustand der Krebszellen angibt, durchgeführt. Eine erfolgreiche Analyse kann nur erfolgen, wenn der Patient heterozygot für beide Allele eines Markers ist. Die Allele unterscheiden sich in ihrer Größe um mindestens 4 bp. Die Quotienten der Peakflächen beider Allele (Allel 1/Allel 2) in der Gesamtblutfraktion werden mit denjenigen der Fraktion C verglichen. Wenn sich die Werte um mindestens 50 % unterscheiden, kann von einem LOH ausgegangen werden.

### Referenzbeispiel 11:

### p53-und K-ras-Mutationsanalysen

### Versuchablauf :

Pro PCR-Ansatz werden folgende Reagenzien zusammengegeben (µl):

| | Cod. 175 | Cod. 245 | Cod. 248 | Cod. 249 | Cod. 273 | k-ras |
|---|---|---|---|---|---|---|
| PCR-Puffer (10x) | 5,0 | 5,0 | 5,0 | 5,0 | 5,0 | 5,0 |
| dNTP-Mix (20 mM) | 0,5 | 0,5 | 0,5 | 0,5 | 0,5 | 0,5 |
| DMSO | - | - | 2,5 | 2,5 | - | - |
| MgCl₂ (25 mM) | - | - | - | - | - | 6,0 |
| Primer A (20 pmol/µl) | 1,0 | 1,0 | 1,0 | 1,0 | 1,0 | 1,0 |
| Primer B (20 pmol/µl) | 1,0 | 1,0 | 1,0 | 1,0 | 1,0 | 1,0 |
| Taq-Polymerase (5U/µl) + Taq-Antikörper 1:1 | 0,5 | 0,5 | 0,5 | 0,5 | 0,5 | 0,5 |
| H₂O | 37,0 | 37,0 | 34,5 | 34,5 | 37,0 | 31,0 |
| DNA | 5,0 | 3,0 | 5,0 | 5,0 | 3,0 | 5,0 |

Die PCR wird auf dem Thermocycler Perkin Elmer 9600 oder 2400 durchgeführt. Folgendes Temperaturprofil wird benutzt:

| | | |
|---|---|---|
| 95°C | 5 min | |
| 94°C | 30 sek | |
| 55°C | 30 sek | |
| 72°C | 30 sek | 37 (für k-ras 35x) |
| 72°C | 5 min | |

Der PCR-Ansatz wird auf 4°C gekühlt.

### Kontrollen:

- Pos. (p53; 248):: DNA der akuten Promyelocytischen Leukämie Zellinie NB-4; DNA der Colonkarzinomzellinien Colo 320
- Pos. (p53; 273):: SW480-Kolonkarzinomzellen
- Pos. (K-ras):: SW480- und SW403-Kolonkarzinomzellen
- Neg. (p53):: Ansatz ohne DNA; Ansatz ohne Restriktion
- Neg. (K-ras):: Normallymphozyten-DNA bzw. DNA einer Negativ-Zellinie

### Primer:

| | Primer A | Primer B |
|---|---|---|
| p53 (175) | FAM-p53 175-S | p53 6-2 |
| p53 (245) | FAM-p53 245-A | p53 245-S |
| p53 (248) | FAM-p53 7-1 | p53 8-2 |
| p53 (249) | FAM-p53 7-1 | p53 8-2TET-p53 8-2 |
| p53 (273) | p53 273-S | TET-p53 8-2 |
| k-ras | k-ras-sense | FAM-markierter K-105-anitsense |

### Analyse der PCR-Produkte:

### 1. Funktionsnachweis der PCR im Agarosegel

9 µl PCR-Produkt und 1 µl 10x TBE-Probenpuffer mit Bromphenolblau werden auf ein 2%iges Agarosegel (10 µl Ethidiumbromid-Stammlösung 10 mg/ml auf 100 ml Gel) in 1 x TBE-Puffer aufgetragen. Die Elektrophoresewird bei 160 bis 170 V und konstanter Spannung durchgeführt.

### 2. Restriktionsverdau

7,3 µl , 2,0 µl 10x Puffer, 0,2 µl 100 x BSA, 0,5 µl Restriktionsenzym und 10 µl PCR-Produkt (für Codon 245, 249 und 273: 7,5 µl H₂O (bidest), 2,0 µl 10x Puffer, 0,5 µl Restriktionsenzym und 10 µl PCR-Produkt; für Codon 248: 7,75 µl H₂O (bidest), 2,0 µl 10x Puffer, 0,25 µl Restriktionsenzym und 10 µl PCR-Produkt) werden 1 Stunde bei 37°C inkubiert. Je nach der Menge des PCR-Produktes erfolgt die Auswertung über ein 4%iges Metaphor-Gel oder mittels ABI 310.

### Auswertung über Metaphor-Agaraosegel:

18 µl restringiertes PCR-Produkt und 2 µl 10x TBE-Probenpuffer mit Bromphenolblau werden auf ein 4%iges Metaphor-Agarosegel ( 10 µl Ethidiumbromid-Stammlösung 10 mg/ml auf 100 ml Gel) in 1 x TBE-Puffer aufgetragen. Die Elektrophorese wird bei 160 bis 170 V und konstanter Spannung durchgeführt. Zum Vergleich wird auf jedes Gel auch das nicht geschnittene PCR-Produkt einer beliebigen Probe aufgetragen.

### Nachweis mittels ABI 310

12 µl Formamid, 1 µl Probe und 0,5 µl Genescan Tamra 500 (PE) werden 2 min bei 95°C denaturiert und direkt auf Eis gestellt. Anschließend wird eine Fragmentanalyse auf dem ABI 310 (Analysepuffer; Anode und Kathode: 1x Fragmentanalysenpuffer (PE) mit EDTA; Polymer: POP-4; Injektionszeit: 5 sec; Injektionsspannung: 15 kV; Spannung während des Laufes: 15 kV; Temperatur während des Laufes: 60°C; Laufzeit: 24 min; Matrix: GS POP4C) durchgeführt.

### Fragmentlängen:

| | | | |
|---|---|---|---|
| **Codon 175** | | | |
| Wildtyp: | **70** bp | 227 bp | 18 bp |
| mutiert: | **88** bp | 227 bp | |
| **Codon 245** | | | |
| Wildtyp: | **145** bp | 23 bp | |
| mutiert: | **168** bp | | |
| **Codon 248** | | | |
| Wildtyp: | **77** bp | | |
| mutiert: | **246** bp | | |
| **Codon 249** | | | |
| Genotyp: | | | |
| Wildtyp: | **81** bp | | |
| mutiert: | **147** bp | | |
| **Codon 273** | | | |
| Wildtyp: | **116** bp | 21 bp | |
| mutiert: | **137** bp | | |
| **K-ras Codon** | **12** | | |
| Wildtyp: | **114** bp | 29 bp | 14 bp |
| mutiert: | **143** bp | 14 bp | |

Zur Auswertung wird das Flächenintegral 88 bp zu 70 bp, 168 bp zu 145 bp, 246 bp zu 77 bp, 147 bp zu 81 bp, 137 bp zu 116 bp bzw. 143 bp zu 114 bp gebildet.

### Beispiel 1 (Diagnostische Fragestellung)

### a) Klinische Ausgangssituation

Bei der zu untersuchenden Patientin bestand eine Mastopathie mit Verdacht auf ein Mammakarzinom. Es wurde eine Blutprobe entnommen.

### b) Fragestellung

Gab es in der entnommenen Blutprobe zirkulierende Krebszellen, die Metastasen bilden konnten?

### c) Untersuchungen und Resultate

| Untersuchung | Ergebnis |
|---|---|
| p53-Mutation in Tumorzellen | NEGATIV |
| p53(Exon5)-Mutation p53(Exon6)-Mutation p53(Exon7)-Mutationen p53(Exon8)-Mutationen | NEGATIV NEGATIV NEGATIV NEGATIV |
| erb-B2 nach Tumorzellisolierung | POSITIV |
| c-myc nach Tumorzellisolierung | POSITIV |
| CK20-mRNA | POSITIV |
| CEA-mRNA | POSITIV |
| MUC1-mRNA | 0.95 |
| MUC1-mRNA Fraktion C | 1.40 |
| bFGF-mRNA | NEGATIV |
| bFGF-R-mRNA | POSITIV |
| VEGF-mRNA | POSITIV |
| VEGF-R1-mRNA | POSITIV |
| VEGF-R2-mRNA | NEGATIV |
| TIMP3-mRNA | NEGATIV |
| MMP2-mRNA | NEGATIV |
| Progesteron-R-mRNA | POSITIV |
| Maspin-mRNA | POSITIV |
| Perforin nach Tumorzellisolierung | NEGATIV |

### d) Bewertung

Bei der Patientin ließen sich hämatogen zirkulierende Zellen nachweisen, die vom Karzinomtyp sein konnten. Krebszellspezifisch konnten Zellen nachgewiesen werden, welche die krebsspezifische splice-Variante des Mucinl-Gens verstärkt transkribierten. Auch CEA- und CK20-mRNA wurde von diesen Zellen exprimiert. Da diese Expressionscharakteristika beim Mammakarzinom angetroffen werden, stammten die Zellen höchstwahrscheinlich aus der Mamma, was durch die Expression von Maspin bestätigt wurde.

Die Tumorzellen ließen sich in der Fraktion C (MUC1) ohne lymphozytäre Verunreinigung (Perforin-negativ) anreichern. Die nachgewiesenen Zellen zeigten auch bereits Anzeichen einer Metastasierungsfähigkeit, da sie den bFGF- und VEGF-Rezeptor sowie VEGF exprimierten, allesamt Prärequisite der Neoangiogenese. Die im Blut vagabundierenden Zellen exprimierten den Progesteron-Rezeptor.

Die c-myc- und erb-B2-Amplifikation sprachen darüber hinaus für eine schlechte Prognose. Qualitativ handelte es sich um einen typischen Hochrisiko-Verlauf mit extremer Wachstumspotenz, da diese Gene für Wachstumssignale im Krebsgewebe kodieren.

### e) Zusammenfassung und Schlußfolgerung:

Insgesamt sprach der Befund für ein streuendes Karzinom mit schlechter Prognose, welches der Mastophatie zugrunde lag.

### Beispiel 2 (Identifikation der Streuquelle)

### a) Klinische Ausgangssituation

Bei dem zu untersuchenden Patienten bestand der Verdacht auf ein Colon- oder Prostatakarzinom. Es wurde eine Blutprobe entnommen.

### b) Fragestellung

Gab es in der entnommenen Blutprobe zirkulierende Krebszellen, die Metastasen bilden konnten? Stammten diese Zellen aus einem Prostata- oder einem Colonkarzinom?

### c) Untersuchungen und Resultate:

| Untersuchung | Ergebnis |
|---|---|
| K-ras nach Tumorzellisolierung | POSITIV |
| K-ras(Exonl)-Mutation K-ras(Exonl)-Mutation | POSITIV NEGATIV |
| CK20-mRNA | POSITIV |
| CEA-mRNA | NEGATIV |
| MUC1-mRNA | > 1,00 |
| Gastrin-mRNA | POSITIV |
| PSM-mRNA | NEGATIV |
| bFGF-mRNA | NEGATIV |
| bFGF-R-mRNA | POSITIV |
| VEGF-mRNA | NEGATIV |
| VEGF-R1-mRNA | NEGATIV |
| VEGF-R2-mRNA | NEGATIV |
| EGP-mRNA | 255370 (entspricht 100%) |
| GAPDH-mRNA | 125836424 (entspricht 100%) |
| EGP-mRNA Faktion C | 1773 (entspricht 0.7% der Fraktion A) |
| GAPDH-mRNA Faktion C | 625 (entspricht 0.0005% der Fraktion A) |

### d) Bewertung

Es konnten hämatogen streuende Zellen nachgewiesen werden, die ausschließlich die tumorspezifische Splice-Variante des MUC1-Gens als auch CK20 transkribierten und daher dem Karzinomtyp zuzuordnen waren. Sie ließen sich in der Fraktion C anreichern. Die hämatogen streuenden Zellen stammten mit Sicherheit aus dem Colon, da Gastrin-mRNA im Gegensatz zur Prostata-spezifischen PSM-mRNA nachgewiesen werden konnte.

In der Epithelzellfraktion C wurde auch das punktmutierte Onkogen k-ras gefunden, wie es für Colonkarzinome typisch ist. Die nachgewiesenen Zellen zeigten ebenfalls bereits Anzeichen einer Metastasierungsfähigkeit, da sie den bFGF-Rezeptor, ein Prärequisit der Neoangiogenese, exprimierten.

### e) Zusammenfassung und Schlußfolgerung:

Insgesamt sprach der Befund für ein streuendes Karzinom, welches sicher colorectal zu suchen war. Eine Therapie mit Panorex schien indiziert, da die Zellen das therapeutische Zielgen EGP exprimierten.

### Beispiel 3 (Prognose)

### a) Klinische Ausgangssituation

Bei der zu untersuchenden Patientin bestand ein malignes Melanom im postoperativen Zustand (Clark II-III).

Es wurde eine Blutprobe entnommen.

### b) Fragestellung

Gab es in der entnommenen Blutprobe zirkulierende Krebszellen, die Metastasen bilden konnten? Konnten weitere prognostische Aussagen zum Tumor gemacht werden?

### c) Untersuchungen und Resultate:

| Untersuchung | Ergebnis |
|---|---|
| p53-Mutation in Tumorzellen | POSITIV |
| pS3(Exon5)-Mutation p53(Exon6)-Mutation p53(Exon7)-Mutationen p53(Exon8)-Mutationen | POSITIV NEGATIV NEGATIV NEGATIV |
| Tyrosinase-mRNA | POSITIV |
| MAGE3-mRNA | POSITIV |
| Muc18-mRNA | POSITIV |
| bFGF-mRNA | NEGATIV |
| bFGF-R-mRNA | POSITIV |
| VEGF-mRNA | POSITIV |
| VEGF-R1-mRNA | POSITIV |
| VEGF-R2-mRNA | NEGATIV |
| MMP2-mRNA | POSITIV |
| FAS-mRNA in Fraktion C | POSITIV |
| FAS-Rezeptor-mRNA in Fraktion C | NEGATIV |
| Perforin nach Tumorzellisolierung | NEGATIV |

### d) Bewertung

Die Resultate sprachen für ein progressives malignes Melanom mit hämatogen streuenden Zellen, die metastasierungsfähig waren. Für die Metastasierungsfähigkeit der im Blut zirkulierenden Zellen sprach die Expression der Angiogenesefaktoren bFGF-Rezeptor, VEGF-Rezeptor 1 und VEGF ebenso wie die Fähigkeit der zirkulierenden Zellen zur Matrixdegradation (MMP2-mRNA nachgewiesen).

In der Fraktion C fand sich eine proteinrelevante Mutation des Onkogens p53 in Exon 5. Die Zellen exprimierten nur FAS-Ligand, nicht aber FAS-Rezeptor; sie waren möglicherweise einer zellvermittelten Cytotoxizität (Apoptose) nicht zugänglich. Eine lymphozytäre Verunreinigung der Fraktion C wurde ausgeschlossen, da sich Perforin nicht nachweisen ließ.

### Beispiel 4 (Therapierelevanz)

### a) Klinische Ausgangssituation

Bei der zu untersuchenden Patientin wurde ein Mammakarzinom diagnostiziert (pT1b N1 M0). Es wurde eine Blutprobe entnommen.

### b) Fragestellung

Gab es in der entnommenen Blutprobe zirkulierende Krebszellen, die Metastasen bilden konnten? Bestand eine Chemoresistenz?

### c) Untersuchungen und Resultate:

| Untersuchung | Ergebnis |
|---|---|
| p53-Mutation in Tumorzellen | POSITIV |
| p53(Exon5)-Mutation p53(Exon6)-Mutation p53(Exon7)-Mutationen p53(Exon8)-Mutationen | NEGATIV NEGATIV NEGATIV POSITIV |
| CK20-mRNA | POSITIV |
| CEA-mRNA | POSITIV |
| MUC1-mRNA | 0.90 |
| MUC1-mRNA Fraktion C | 1.40 |
| bFGF-mRNA | NEGATIV |
| bFGF-R-mRNA | POSITIV |
| VEGF-mRNA | POSITIV |
| VEGF-R1-mRNA | POSITIV |
| VEGF-R2-mRNA | NEGATIV |
| Maspin-mRNA | POSITIV |
| MDR1-Efflux-Doxorubicin-Test | 55 % (Ref.: 40 - 65 %) |
| MDR1-Pumpe-gp170 | 61 % (Ref.: 40 - 65 %) |
| MDR1 (menschliches Genom) | NEGATIV |
| MDR-mRNA Fraktion C | NEGATIV |
| MRP-mRNA Fraktion C | NEGATIV |
| GST-pi-mRNA Fraktion C | NEGATIV |
| Topoisomerase-II-mRNA Fraktion C | POSITIV |

### d) Bewertung

Bei der Patientin ließen sich hämatogen zirkulierende Zellen nachweisen, die vom Karzinomtyp sein konnten. Krebszellspezifisch konnten Zellen nachgewiesen werden, welche die krebsspezifische Splice-Variante des Mucinl-Gens verstärkt transkribierten. Auch CEA- und CK20-mRNA wurde von diesen Zellen exprimiert. Da diese Expressionscharakteristika beim Mammakarzinom angetroffen werden, stammten die Zellen höchstwahrscheinlich aus der Mamma, was durch die Expression von Maspin bestätigt wurde.

Die nachgewiesenen Zellen zeigten auch bereits Anzeichen einer Metastasierungsfähigkeit, da sie den bFGF- und VEGF-Rezeptor sowie VEGF exprimierten, allesamt Prärequisite der Neoangiogenese. Außerdem ließ sich in der Tumorzellfraktion eine p53-Mutation in Exon 8 nachweisen. Dies ist ebenfalls ein für das Mammakarzinom typischer Befund.

### e) Zusammenfassung und Schlußfolgerung:

Insgesamt sprach der Befund für ein metastasierungsfähiges Karzinom. Bei der Patientin bestand kein Hinweis auf eine Chemoresistenz. Aufgrund der nachgewiesenen Topoisomerase-Expression als Zielgen schien eine Antrazyklintherapie indiziert zu sein.

### Beispiel 5 (vor Chemotherapie)

### a) Klinische Ausgangssituation

Bei der zu untersuchenden Patientin wurde ein Mammakarzinom diagnostiziert. Die Patientin stand nicht unter Chemotherapie. Es wurde eine Blutprobe entnommen.

### b) Fragestellung

Gab es in der entnommenen Blutprobe zirkulierende Krebszellen, die Metastasen bilden konnten? Bestand eine Chemoresistenz?

### c) Untersuchungen und Resultate:

| Untersuchung | Ergebnis |
|---|---|
| p53-Mutation in Tumorzellen | NEGATIV |
| p53(Exon5)-Mutation p53(Exon6)-Mutation p53(Exon7)-Mutationen p53(Exon8)-Mutationen | NEGATIV NEGATIV NEGATIV NEGATIV |
| erb-B2 nach Tumorzellisolierung | NEGATIV |

| c-myc nach Tumorzellisolierung | NEGATIV |
|---|---|
| CK20-mRNA | POSITIV |
| CEA-mRNA | POSITIV |
| MUC1-mRNA | 0.55 |
| bFGF-mRNA | NEGATIV |
| bFGF-R-mRNA | NEGATIV |
| VEGF-mRNA | NEGATIV |
| VEGF-R1-mRNA | NEGATIV |
| VEGF-R2-mRNA | NEGATIV |
| MDR1-Pumpe-gp170 | 18 %¹⁾ (Ref.: 40 - 65 %) |
| MDR1 (menschliches Genom) | NEGATIV |
| MDR1-mRNA | POSITIV |
| GST-pi-mRNA | POSITIV |
| Topoisomerase-II-mRNA | POSITIV |
| MRP-mRNA | POSITIV |
| EGP-mRNA | 30743 (entspricht 100 %) |
| GAPDH-mRNA | 1765600 (entspricht 100 %) |
| EGP-mRNA Fraktion C | 1 |
| GAPDH-mRNA Fraktion C | 0 |
| MUC1-mRNA Fraktion C | > 1,00 |
| bFGF-mRNA Fraktion C | 0,00 |

| | |
|---|---|
| 1) Unspezifische Antikörper-Bindungskapazität (ABC): 953 Spezische ABC: 2935 | |

### e) Bewertung

Bei der Patientin ließen sich hämatogen zirkulierende vom Karzinomtyp nachweisen, welche die krebsspezifische Splice-Variante des MUC1-Gens transkribierten. Auch die Expression von CEA und CK20 konnte nachgewiesen werden. Diese Expressionscharakteristika sprachen für ein Mammakarzinom. Da die Analyse der obigen Angiogenesefaktoren negativ ausfiel, konnte davon ausgegangen werden, daß die zirkulierenden Krebszellen nur ein geringes Metastasierungspotential zeigten. Bei der Patientin bestand kein Hinweis auf eine Chemoresistenz.

### e) Zusammenfassung und Schlußfolgerung:

Insgesamt sprach der Befund für ein streuendes Mammakarzinom ohne Anzeichen einer Chemoresistenz.

### Beispiel 6 (nach Chemotherapie)

### a) Klinische Ausgangssituation

Bei der Patientin handelte es sich um dieselbe, die im Beispiel 5 beschrieben wurde. Aufgrund des gemäß Beispiel 5 gestellten Befundes wurde die Patientin mit einer adjuvanten Chemotherapie und mit Tamoxifen behandelt. Nach Abschluß der Chemotherapie wurde der Patientin erneut Blut entnommen.

### b) Fragestellung

Gab es in der entnommenen Blutprobe zirkulierende Krebszellen, die Metastasen bilden konnten?

### c) Untersuchungen und Resultate:

| Untersuchung | Ergebnis |
|---|---|
| anti-p53 | niedrig |
| Pan p53 | 243 pg/ml normal bis 646 grenzwertig bis 786 pathologisch ab 787 |
| c-erb-B2 | 2610 HNU/ml normal bis 3385 grenzwertig 3386 - 3845 pathologisch >3845 |
| p53-Mutation in Tumorzellen | NEGATIV |
| p53(Exon5)-Mutation p53(Exon6)-Mutation p53(Exon7)-Mutationen p53(Exon8)-Mutationen | NEGATIV NEGATIV NEGATIV NEGATIV |
| erb-B2 nach Tumorzellisolierung | NEGATIV |
| c-myc nach Tumorzellisolierung | NEGATIV |
| CK20-mRNA | NEGATIV |
| CEA-mRNA | NEGATIV |
| MUC1-mRNA | 0.60 |
| bFGF-mRNA | NEGATIV |
| bFGF-R-mRNA | POSITIV |
| VEGF-mRNA | schwach positiv |
| VEGF-R1-mRNA | POSITIV |
| VEGF-R2-mRNA | NEGATIV |
| TIMP3-mRNA | NEGATIV |
| MMP2-mRNA | NEGATIV |
| Progesteron-R-mRNA | POSITIV |
| EGP-mRNA | 232255 (entspricht 100 %) |
| GAPDH-mRNA | 463040096 (entspricht 100 %) |
| EGP-mRNA Fraktion C | 0 |
| GAPDH-mRNA Fraktion C | 203 (entspricht 0,00004 % von Fraktion A) |
| MUC1-mRNA Fraktion C | 0.00 |

### d) Bewertung

Bei der Patientin ließen sich hämatogen zirkulierende Zellen nachweisen, die vom Karzinomtyp sein konnten.

Krebszellspezifisch konnten Zellen nachgewiesen werden, welche die krebsspezifische Splice-Variante des Mucinl-Gens verstärkt transkribierten. Die nachgewiesenen Zellen zeigten erste Anzeichen einer Metastasierungsfähigkeit; sie exprimierten bFGF-R-, VEGF- und VEGF-R1-mRNA; MMP-2 konnte jedoch nicht nachgewiesen werden. Die im Blut vagabundierenden Zellen waren Progesteron-Rezeptor positiv. Unter den prognostischen Onkoproteinen war anti-p53 nachweisbar; dieses durch p53-Mutationen reaktive Protein deutete auf eine reduzierte Prognose hin.

### e) Zusammenfassung und Schlußfolgerung:

Insgesamt sprach der Befund für ein noch streuendes Karzinom. Im Vergleich zum Vorbefund (vor der Chemotherapie) lag jedoch ein deutlich verändertes Bild vor. So war insbesondere MUC1 in der Fraktion C nicht mehr nachzuweisen und in der Fraktion A waren die Marker CK20 und CEA negativ. Dies sprach für einen deutlichen Therapieerfolg.

### Beispiel 7

### a) Klinische Ausgangssituation

Bei der zu untersuchenden Patientin bestand ein Verdacht auf Ovarkarzinom. Es wurde eine Blutprobe entnommen.

### b) Fragestellung

Gab es in der entnommenen Blutprobe zirkulierende Krebszellen, die Metastasen bilden konnten? Bestand eine Chemoresistenz?

### c) Untersuchungen und Resultate

| Untersuchung | Ergebnis |
|---|---|
| anti-p53 | NEGATIV |
| Pan p53 | 440 pg/ml normal bis 646 grenzwertig bis 786 pathologisch ab 787 |
| c-erb-82 | 2641 HNU/ml normal bis 3385 grenzwertig 3386 - 3845 pathologisch >3845 |
| p53-Mutation in Tumorzellen | NEGATIV |
| p53(Exon5)-Mutation p53(Exon6)-Mutation p53(Exon7)-Mutationen p53(Exon8)-Mutationen | NEGATIV NEGATIV NEGATIV NEGATIV |
| erb-B2 nach Tumorzellisolierung | NEGATIV |
| c-myc nach Tumorzellisolierung | NEGATIV |
| CK20-mRNA | NEGATIV |
| CEA-mRNA | NEGATIV |
| MUC1-mRNA | 0.35 |
| bFGF-mRNA | NEGATIV |
| bFGF-R-mRNA | NEGATIV |
| VEGF-mRNA | nicht bewertbar |
| VEGF-R1-mRNA | NEGATIV |
| VEGF-R2-mRNA | NEGATIV |
| Progesteron-R-mRNA | schwach positiv |
| MDR1-mRNA | POSTIV |
| Topoisomerase II-mRNA | NEGATIV |
| MDR1-Efflux-Doxorubicin-Test ¹⁾ | 0 % |
| MDR1-Pumpe-gp170²⁾ | 0 % |
| Glutation-S-Transferase-mRNA | POSITIV |
| MRP-mRNA | POSITIV |

| | |
|---|---|
| ¹⁾ Bemerkung zum Parameter: Im Gegensatz zu den Kontrollzellen konnten die Lymphozyten kein Doxorubicin akkumulieren. Damit war die Bestimmung des Effluxes nicht möglich. ²⁾ Bemerkung zum Parameter: Gegenüber den positiven Kontrollzellen ließ sich keine Expression der MDR1-Pumpe-gp170 auf den Lymphozyten nachweisen. Die quantitative Analyse ergab folgendes: Unspezifische Antikörper-Bindungskapazität (ABC): 1523 Spezifische ABC: 4165 | |

### d) Bewertung

Bei der Patientin ließen sich hämatogen zirkulierende Krebzellen vom Karzinomtyp nachweisen, welche die krebsspezifische Splice-Variante des MUC1-Gens verstärkt transkribierten.

Da die Analysen obiger Angiogenesefaktoren negativ ausfiel, konnten keine Zellen nachgewiesen werden, die zur Neoangiogenese, dem funktionellen Zusammenspiel von Endothel- und Epithelzellen, befähigt waren. Die zirkulierenden Krebszellen zeigten daher keine Metastasierungsfähigkeit; von der Bildung aktiver Metastasen war nicht auszugehen. Die im Blut zirkulierenden Zellen exprimierten den Progesteron-Rezeptor. Bei der Patientin bestand kein Hinweis auf eine Chemoresistenz.

### e) Zusammenfassung und Schlußfolgerung

Insgesamt sprach der Befund möglicherweise für ein Karzinom eines hormonsensitiven Organs.

### Beispiel 8:

### a) Klinische Ausgangssituation

Bei der zu untersuchenden Patientin bestand aufgrund eines suspekten Mammabefundes (walnußgroßer Tumor bei unauffälliger Mammographie) ein Verdacht auf Mammakarzinom. In der Familie waren drei Fälle von Mamma-Neoplasien, davon zwei bei Patientinnen unter 50 Jahren und ein Fall einer Ovar-Neoplasie bekannt. Es bestand daher eine erbliche Tumor-Disposition. Es wurde eine Blutprobe entnommen.

### b) Fragestellung

Gab es in der entnommenen Blutprobe zirkulierende Krebszellen, die Metastasen bilden konnten?

### c) Untersuchungen und Resultate

| Untersuchung | Ergebnis |
|---|---|
| anti-p53 | NEGATIV |
| Pan p53 | 87 pg/ml normal bis 646 grenzwertig bis 786 pathologisch ab 787 |
| c-erb-B2 | 2540 HNU/ml normal bis 3385 grenzwertig 3386 - 3845 pathologisch >3845 |
| p53-Mutation in Tumorzellen | NEGATIV |
| p53(Exon5)-Mutation p53(Exon6)-Mutation p53(Exon7)-Mutationen p53(Exon8)-Mutationen | NEGATIV NEGATIV NEGATIV NEGATIV |
| erb-B2 nach Tumorzellisolierung | NEGATIV |
| c-myc nach Tumorzellisolierung | NEGATIV |
| CK20-mRNA Fraktion A | POSITIV |
| CEA-mRNA Fraktion A | schwach positiv |
| MUC1-mRNA Fraktion A | 0.86 |
| bFGF-mRNA Fraktion A | POSITIV 909 entspricht 100% (Fraktion A) |
| bFGF-R-mRNA Fraktion A | NEGATIV |
| VEGF-mRNA Fraktion A | POSITIV 72437 entspricht 100% (Fraktion A) |
| VEGF-R1-mRNA Fraktion A | NEGATIV |
| VEGF-R2-mRNA Fraktion A | NEGATIV |
| TIMP3-mRNA Fraktion A | POSITIV |
| MMP2-mRNA Fraktion A | schwach positiv |
| Progesteron-R-mRNA | NEGATIV |
| EGP-mRNA Fraktion A | 104097 (entspricht 100 %) |
| GAPDH-mRNA Fraktion A | 153350963 (entspricht 100 %) |
| CK20-mRNA Fraktion C | NEGATIV |
| CEA-mRNA Fraktion C | NEGATIV |
| MUC1-mRNA Fraktion C | 0,00 |
| bPGF-mRNA Fraktion C | 0,00 |
| VEGF-mRNA Fraktion C | 1738 (2,4 % bezogen auf Fraktion A) |
| TIMP3-mRNA Fraktion C | NEGATIV |
| MMP2-mRNA Fraktion C | NEGATIV |
| EGP-mRNA Fraktion C | 0 |
| GAPDH-mRNA Fraktion C | 108644 (entspricht 0,00708 % von Fraktion A) |

### d) Bewertung

Bei der Patientin ließen sich geringe Mengen an hämatogen zirkulierenden Krebzellen vom Karzinomtyp nachweisen, welche die krebsspezifische Splice-Variante des MUC1-Gens verstärkt transkribierten. Auch die Expression von CK20 und CEA konnte nachgewiesen werden. Diese Expressionscharakteristika sprachen für ein Mammakarzinom. Die zirkulierenden Krebszellen konnten allerdings nicht angereichert werden.

Da die Analysen obiger Angiogenesefaktoren teilweise positiv ausfielen (Expression von bFGF und VEGF), konnten Zellen nachgewiesen werden, die zur Neoangiogenese, dem funktionellen Zusammenspiel von Endothel- und Epithelzellen, befähigt waren. Die zirkulierenden Krebszellen zeigten daher bereits Anzeichen einer Metastasierungsfähigkeit. Dafür sprach auch die Expression des MMP2-Gens. Die Bildung aktiver Metastasen war daher sehr wahrscheinlich. Der Progesteron-Rezeptor wurde von den im Blut zirkulierenden Zellen nicht exprimiert.

### e) Zusammenfassung und Schlußfolgerung

Insgesamt sprach der Befund für ein streuendes Karzinom. Die zirkulierenden Zellen stammten aus der Mamma.

### GLOSSAR

### AFP (Alpha-Fetoprotein)

AFP ist das Hauptplasmaprotein im Fetus. Im Adulten ist die Expression von AFP sehr gering, es sei denn es liegt ein Tumor, wie ein Hepatom oder ein Teratom, vor.

**Lit.:** Gibbs et al.; Biochemistry 26: 1332-1343, 1987.

### β-Aktin

**Lit.:** Pollard, T.D. and Cooper, J.A.; Ann. Rev. Biochem. 55, 987 ff, 1986.

### Albumin (ALB)

Albumin dient zur Identifizierung von im Blut zirkulierenden Hepatomzellen.

**Lit.:** Minghettei et al.; J. Biol. Chem. 261: 6747-6757, 1986.

### AR (Androgen-Rezeptor)

Synonyme: Dihydrotestosteron-Rezeptor, Testosteron-Rezeptor, TFM

Prostatakarzinomzellen sind von der wachstumsstimulierenden Wirkung des AR abhängig. In Prostatakarzinomen konnten Mutationen des Androgen-Rezeptors nachgeweisen werden, die teilweise zu einem konstitutiv aktiven Rezeptor führen.

**Lit.:** Lubahn et al.; Proc. Natl. Acad. Sci. USA 87(11): 9534-9538, 1989.

### BA46 (Breast Epithelial Antigen 46)

Synonym: Human Milk Fat Globule Protein

Das Glykoprotein BA-46 wird von Mammakarzinomen exprimiert und wurde erfolgreich als Ziel experimenteller Radioimmunotherapien benutzt.

**Lit.:** Couto et al.; DNA Cell Biol. 15: 281-286, 1996.

### Basischer Fibroblastenwachstumsfaktor (bFGF)

Synonym: FGF-2

Bei vielen Tumorarten wird bFGF überexprimiert und kann daher als ein Faktor für die Metastasierungsfähigkeit angesehen werden.

**Lit.:** Abraham et al.; EMBO J. 5: 2523-2528, 1986.

### BAX

Das bcl-2-Produkt heterodimerisiert in-vivo mit einem konservierten Homologen, dem BAX, wodurch der programmierte Zelltod beschleunigt wird.

**Lit.:** Tsujiimoto Y. and Croce C.M.; PNAS, 83 (14), 5214-5218, 1986.

### bcl-2

Das bcl-2-Gen wurde in follikulären Non-Hodgkin-Lymphomen (B-Zell-Lymphomen) entdeckt. BCL-2 kann die Apoptose blockieren.

**Lit.:** Tsujiimoto Y. and Croce C.M.; PNAS, 83 (14), 5214-5218, 1986.

### BRCA1

Das BRCA1-Gen ist ein Tumorsuppressor-Gen. Bei 5-10% der Patientinnen mit Brustkrebs besteht eine erbliche Disposition, womit häufig auch eine Disposition für Ovarkarzinome verbunden ist. Mutationen des BRCA1-Gens stehen in Zusammenhang mit 45% der Mammakarzinome mit erblicher Komponente. Mutationen im BRCA1-Gen betreffen auch Ovarkarzinome.

**Lit.:** Smith T.M., et al.; Genome Res. 6, 1029-1049, 1996.

### BRCA2

Das BRCA1-Gen ist ein Tumorsuppressor-Gen. Mutationen dieses Gens werden für einen hohen Anteil früh entstehender erblicher Brusttumoren verantwortlich gemacht.

**Lit.:** Lancaster J.M., et al.; Nature Genet. 13, 238-240, 1996.

### Calcitonin

Calcitonin (32 Aminosäuren) wird wie "Calcitonin gene-related peptide (CGRP; 37 Aminosäuren)" von einem Gen kodiert, dem calc-1. Calcitonin kann das Wachstum einer gastrischen Karzinomzellinie hemmen und das Neurohormon CGRP kann als autokriner Wachstumsfaktor für murine Karzinomzellinien fungieren.

Lit.: Adema and Baas; BBRC 178: 985-992, 1991.

### CC10 (Clara cell 10 kD Protein)

CC10 wird nur in Alveolarepithelzellen Typ 2 und in Clara-Zellen des Lungenepithels exprimiert und ist an der Bildung der "epithelial lining fluid" beteiligt.

**Lit.:** Am. L. Physiol. 268: L565 (1995).

### CCK (Cholecystokinin)

CCK ist ein Gehirn- und Darmhormon. CCK wird zudem von einigen Sarkom-Neuroepitheliomas-Zellinien exprimiert.

Lit.: Friedman, J.M. et al.; PNAS 89: 5819-5823, 1992.

### CD44

Synonyme: Hermes-Antigen, Pgp-1

Das CD44-Glykoprotein ist ein Zelladhäsionsmolekül. Bestimmte Splicevarianten von CD44 spielen eine Rolle im Metastasierungsprozeß von Tumoren.

Lit.: Matsumura, Y. and Tarin, D.; The Lancet 340, 1053-1058, 1992.

### CEA (carcinoembryonalic antigen)

Synonym: CD66e

CEA wird in gastrointestinalen und colorectalen Karzinomen, aber auch in verschiedenen soliden Tumoren, wie Mammakarzinomen, im fetalen Colon, nicht aber in normalen Lymphozyten exprimiert wird. Wegen dieses Expressionsprofils dient der Nachweis CEA-positiver Zellen im Blut zur Diagnose zirkulierender Tumorzellen. Weiterhin sind CEA-Immunoassays wichtige Diagnoseverfahren bei der Beobachtung von Krebs-Patienten, besonders im Fall von Colonkarzinomen.

Lit.: Zimmermann et al.; Proc. Natl. Acad. Sci. USA 84: 2960-2964, 1987.

### CK20 (Cytokeratin 20)

Maligne Zellen behalten in der Regel das Muster an Cytokeratinen und dieses kann demgemäß zur Rücklokalisation der Tumorzellen auf ein Epithel benutzt werden. Da CK20 nicht von Zellen des peripheren Blutes exprimiert wird, sondern hauptsächlich von Zellen des Gastrointestinaltraktes, dient dieses Cytokeratin zum Nachweis im Blut zirkulierender Tumorzellen dieses Ursprungs.

Lit.:Moll et al.; Differentiation 63: 75-93, 1993; Burchill et al.; British Journal of Cancer 71:278-281, 1995.

### Cyclin A, B(1), D1, D2, D3, und E

Cycline und Cyclin-abhängige Kinasen (CDK) sind essentiell für die Kontrolle des Zellzyklus eukaryotischer Zellen. Die Messung der Cycline korreliert mit dem Zellzyklus.

Lit.: Motokura T., et al.; J. Biol. Chem. 1992 Oct 5; 267(28): 20412-5;

Lees E., et al.; Genes-Dev. 1992 Oct; 6(10): 1874-85.

### Cyclin G

Cyclin G1 und Cyclin G2 stellen zwei erst kürzlich identifizierte Cycline dar, welche im Zellzyklus eine Rolle spielen.

Lit.: Horne M.C., et al.; J. Biol. Chem. 1996 Mar 15; 271(11): 6050-61.

### DCC

In colorectalen Tumoren sind häufig Sequenzen aus Chromosom 18 deletiert (DCC = Deleted in colorectal carcinomas). Die Expression des DCC-Gens ist in den meisten colorectalen Karzinomen stark reduziert. Der Verlust der 18q-Region ist mit einer schlechten Prognose verbunden. Der Status des DCC-Gens kann mittels Mikrosatelliten-Markern und PCR aus Formalin-fixiertem Material bestimmt werden.

**Lit.:** Frank C.J., et al.; Cancer Res. 57, (5), 824-827, 1997.

### DPC4

Die Bezeichnung bedeutet "deleted in pancreatic carcinoma". Etwa 90% der humanen Pankreaskarzinome zeigen einen Allelverlust auf Chromosom 18. Es handelt sich um ein Tumorsuppressor-Gen. Auch in Brust- und Ovarkarzinomen wurden Änderungen des DPC4-Gens entdeckt.

**Lit.**: Hahn S.A., et al.; Science 271, 350-354, 1996.

### E-Cadherin

### catenin (α- und β-)

E-Cadherin ist, in Verbindung mit assoziierten Cateninen (α-Catenin; β-Catenin) für die Organogenese und Histogenese von Epithelgewebe wichtig und spielt im Metastasierungsprozeß von Karzinomen eine zentrale Rolle.

Lit.: Aberle H., et al.; J. Cell. Biochem. 1996 Jun 15; 61(4): 514-23.

### EGF (Epidermaler Wachstumsfaktor)

Synonyme: HMGF (human milk growth factor); PGF (prostatic growth factor); Urogastron

EGF ist an der embryonalen Entwicklung beteiligt (ektodermale, mesodermale und endodermale Zellen) und kontrolliert/stimuliert die Proliferation von epidermalen und epithelialen Zellen in-vitro. EGF kann ebenfalls als angiogener und chemotaktischer Faktor wirken.

Lit.: Carpenter: EGF; Curr. Opin. Cell. Biol. 5: 261-264, 1993.

### EGF-R (EGF-Rezeptor)

Synonym: SA-7 (species antigen 7)

In einigen humanen Tumoren wird der EGF-R überexprimiert, was auch mit der Tumoraggressivität korreliert; eine schlechte Prognose ergibt sich aus der Coexpression des EGF-R mit entweder c-erb-B2 oder TGF-alpha.

**Lit.:** Ibelgaufts: Dictionary of cytokines, VCH, 1994.

### EGP (Epitheliales Glykoprotein)

Synonyme: GA733-2; 17-1A-Antigen; KS1/4

Das epitheliale Glykoprotein kann als epithelspezifischer Marker zum Nachweis von Karzinomen dienen.

**Lit.**: Simon, B. et al.; PNAS 87: 2755-2759, 1990; Szala, S. et al.; PNAS 87: 3542-3546, 1990.

### Enteroglucagon

Synonyme: EG, Glucagon 37

Enteroglucagon ist ein Peptid, welches von den Zellen des Jejuno-Ileums produziert wird.

Lit.: Bell, G.I. et al.; Nature 304: 368-371, 1983.

### erb-B

Das erb-B- Gen kodiert für den Rezeptor (EGF-R) des epidermalen Wachstumsfaktors (EGF). Dieses Gen ist in etwa 50% der fortgeschrittenen humanen Glioblastomen amplifiziert.

**Lit.:** Haley J., et al.; Oncogene Res. 1, 375-396, 1987.

### erb-B2

Synonyme: c-erb-B2; avian erythroblastic leukemia viral oncogene homolog 2; NGL (neuroblastoma or glioblastomaderived); neu; tyrosine kinase-type cell surface receptor HER2; TKR1

Erb-B2 kodiert ein Tumorantigen, P185, welches serologisch mit dem Epidermalen Wachstumsfaktor-Rezeptor (EGF-R) verwandt ist. Eine Überexpression konvertiert das Gen für einen normalen Wachstumsfaktor-Rezeptor, erb-B2, in ein Onkogen. Eine Amplifikation von erb-B2 wird in Adenokarzinomen, bei Brust- und Ovarkrebs beobachtet. Erb-B2 ist außerdem bei der Entwicklung von akuter promyeloischer Leukämie (APL) involviert, da das Gen im Band q21.1 von Chromosom 17 lokalisiert ist, wo sich auch der Bruchpunkt der Translokation zwischen Chromosom 15 und 17 befindet (t15:17).

**Lit.:** Slamon et al.; Science 244: 707-712, 1989.

### FAP (APC)

Das Gen der familiären adenomatösen Polyposis coli, einer autosomal-dominanten Erkrankung, ist das fap.

**Lit.:** Groden j. et al.; Cell 66: 589-0, 1991.

### FAS-R; FAS-L (CD95, CD95-L)

FAS gehört in die Gruppe der Apoptose-auslösenden Faktoren.

**Lit.:** Alderson M.R.; J. Exp. Med. 181, (1), 71-77, 1995; Itoh N.; Cell 66, (6), 233-243, 1991.

### FGF-Rezeptoren

Synonyme: fms-like tyrosine kinase-2; FLT2; FMS-like gene; FLG (bFGF-R1); K-SAM, bek (FGF-R2)

Unterschiedliche Expression und alternatives Splicen können in der malignen Progression von Tumoren kritisch sein.

**Lit.:** Yamaguchi et al.; Proc. Natl. Acad. Sci. USA 91: 484-488, 1994.

### c-fos

Den c-fos- und c-jun-Genen kommt ein zentraler Stellenwert im Rahmen der Wachstumsregulation zu.

**Lit.:** Ekstrand A.J., et al.; Exp. Cell. Res. 169. 262-266, 1987.

### GADD45

Gadd45 ist ein Wachstumsarrest- und ein durch DNA-Schäden induziertes Gen, welches durch das p53-Tumorsuppressor-Gen reguliert wird.

**Lit.:** Constance, C.M. et al.; Mol-Cell-Biol. 1996 Jul; 16(7): 3878-83

Crawford, D.R. et al.; Arch-Biochem-Biophys. 1996 May 15; 329(2): 137-44.

### GAPDH (Glyceraldehyde-3-phosphate-Dehydrogenase)

Dieses Gen wird in allen Zellen exprimiert. Die Expression dieses Gens korreliert mit der Zellzahl und dient zur quantitativen und qualitativen Bestimmung der cDNA.

Lit.: Allen, R.W. et al.; J. Biol. Chem. 262 (2), 649-653, 1987.

### Gastrin (GAS)

Gastrin wird vornehmlich von mucosalen Zellen des Magens und den D-Zellen im Pankreas produziert.

**Lit.:** Boel, E. et al.; PNAS 80: 2866-2869, 1983.

### GD-AIF (Glioma-Derived Angiogenesis Inhibitory Factor)

Das GD-AIF gehört ebenso wie Thrombospondin und Angiostatin zu den endogenen Negativ-Regulatoren der Angiogenese. Das Ausmaß, in dem die negativen Regulatoren während der Umschaltphase auf den angiogenischen Phenotyp der Tumorgenese abnehmen, entscheidet darüber, ob ein Primärtumor langsam oder schnell wächst und ob Metastasen gebildet werden.

**Lit..:** Folkman J.; Nat. Med. 1 (1995) 27-31.

### GIP (Gastric Inhibitory Polypeptide)

Synonym: Glucose-Dependent Insulinotropic Polypeptide Produziert wird dieses Hormon vornehmlich von Zellen des oberen Dünndarms.

**Lit.:** Inagaki, N. et al.; Molec. Endocr. 3: 1014-1021, 1989.

### GST-pi (Glutathion-S-Transferase-pi)

GST-pi kodiert für ein detoxifizierendes Enzym und spielt daher bei der Entwicklung von chemoresistenten Tumoren eine Rolle. Nach Chemotherapie konnte eine Steigerung der Expression in Tumoren beobachtet werden, was mit einer ungünstigen Prognose und Chemoresistenz einhergeht.

**Lit.:** Morrow et al.; Gene 75: 3-11, 1989.

### Granzym

Die Hauptfunktion der Granzyme liegt in der Lyse von Tumorzellen und virusinfizierten Zellen durch eine apoptotische Fragmentierung der DNA.

**Lit.:** Kummer, J.A. et al.; Kidney Int. 47: 70-77 (1995).

### hCG (Humanes Chorionisches Gonadotropin)

Die β-Untereinheit des hCG dient als Marker für Keimbahntumoren und Chorionkarzinome und die Detektion der hCG-mRNA mittels RT-PCR ist auch in der Diagnostik metastasierender Mammakarzinome und maligner Melanome nützlich

**Lit.:** Doi F. et al; Int. J. Cancer 65 (1996) 454-459.

### HIC-1 (hypermethylated in cancer)

Das HIC-1 gilt als mögliches Tumorsuppressor-Genprodukt. In Tumorzellen, in denen es hypermethyliert ist, erfolgt eine Unterexpression.

**Lit.:** Wales M. M., et al.; Nat.Med. 1 (1995) 570-577.

### HSP70

Hitzeschockproteine wie HSP70 können bei "Escape-Mechanismen" von Tumorzellen eine Rolle spielen.

Lit.: Kaur J. and Ralhan R.; 63(6): 774-9.

### hTG (humanes Thyreoglobulin)

hTG ist ein Schilddrüsenprotein. Es wurden vier Transkripte identifiziert, welche die Folge alternativen Splicens sind.

**Lit.:** Bertaux et al.; Gene 156: 297-301, 1995.

### ICAM (Interzelluläre Adhäsionsmoleküle)

Synonyme: ICAM-1 (CD54, ICAM1-1); ICAM-2 (CD102); ICAM-3 Die ICAM's-1, -2, und -3 sind Zelloberflächenmoleküle und dienen als Liganden der Leukozytenintegrine.

### IGF (Insulin-like growth factor)

Synonyme: MSA (multiplication-stimulating activity); Somatomedin; NSILA (non-suppressible insulin-like activity); SF (sulfation factor), SFA, SGF (Skeletal growth factor), SMP IGF's wirken als mitogene, autokrine und angiogene Faktoren.

**Lit.:** Cohick and Clemmons; Annual Review of Physiology 55: 131-153, 1993.

### IGF-BP3 (Insulin-like growth factor binding protein 3)

Synonyme: IGBP; IBP; BP-53; growth hormone dependent binding protein; binding protein 29

IGF-BP3 fungiert als Wachstumsinhibitor.

**Lit.:** Lamson; Growth factors 5: 19-28, 1991.

### Integrine

Integrine sind heterodimere Zelloberflächenantigene, die an Zell-Zell- und Zell-Matrix-Wechselwirkungen beteiligt sind.

**Lit.:** VIth International Human Leukocyte Differentiation Antigen Workshop and Conference, Kobe (Japan), November 1996.

### Interferon-gamma

Synonyme: Immune interferon; type 2 interferon; T interferon; antigen-induced interferon; mitogen-induced interferon; ph2-labile interferon

**Lit.:** Gray et al.; Nature 295: 503-508, 1982; Ibelgaufts: Dictionary of cytokines. VCH 1994.

### LOH's

Die Inaktivierung von Tumorsuppressorgenen ist ein kritischer Schritt in der Tumorentstehung. Häufige Mechanismen sind sowohl inaktivierende Mutationen, sowie der genomische Verlust des gesamten Gens, oder Teilen des Gens. Der genomische Verlust von Chromosomenabschnitten kann durch den "Verlust der Heterozygotie" (Loss of Heterozygosity = LOH) experimentell nachvollzogen werden. Dabei finden sich im Normalgewebe eines Patienten beide Allele des Tumorsupressorgens, während im Tumor nur noch ein Allel nachweisbar ist. Zur Identifizierung der zwei Allele dienen im oder in der Nähe des Tumorsuppressorgens lokalisierte hochpolymorphe chromosomale Bereiche (Mikrosatelliten repeats), die durch eine PCR amplifiziert werden. Hierbei handelt es sich um Wiederholungen kurzer Nucleotidabfolgen (zb. CA-Repeats, CGG Repeats), wobei die Kopienzahl unterschiedlich ist und damit das in der PCR amplifizierte Produkt unterschiedliche Längen aufweist.

### L32

Aufgrund der ubiquitären Expression von L32 eignet es sich als Zielgen für Quantifizierungen.

Lit.: Young, J.A. and Trowsdale, J.; Nucleic Acids Res. 13 (24), 8883-8891, 1985.

### LRP

Synonyme: Protein-Tyrosinephosphatase; Alpha-Polypeptide; PTPRA; PTPA

Lrp kodiert für eine ubiquitär exprimierte Protein-Tyrosin-Kinase.

**Lit.:** Jirik et al.; FEBS Lett. 273: 239-242, 1990.

### MAGE1 (Melanoma associated antigen-1)

### Synonym: MZ2-E

Das MAGE1-Gen kodiert für ein Antigen auf der Oberfläche von Melanom-Zellen. Während MAGE1 auf der RNA-Ebene in vielen Tumoren auf hohem Level nachweisbar ist, findet sich die RNA nicht in normalen Geweben mit der Ausnahme von Hoden und Ovar. Dieses Genprodukt ist also hervorragend als Marker für zirkulierende Tumorzellen, insbesondere Melanom-Zellen, geeignet.

**Lit.:** De Plaen et al.; Immunogenetics 40: 360-369, 1994.

### MAGE3 (Melanoma associated antigen-3)

Das MAGE3-kodierende Gen wird in etwa 69% der Melanome transkribiert. Da es bisher nur in Tumorgewebe und bis auf Hoden in keinem Normalgewebe gefunden wurde, eignet sich dieses Gen als Marker für zirkulierende Melanom-Zellen.

**Lit.:** Gaugler et al.; J. Exp. Med. 179: 921-930, 1994.

### Maspin

Bei Maspin handelt es sich um ein Tumorsuppressor-Gen. Defekte dieses Gens finden sich insbesondere in Mammakarzinomzellen.

**Lit.:** Luppi et al.; Annals of Oncol. 7: 619-624, 1996.

### mdm2

Der onkogene Effekt einer gesteigerten MDM2-Aktivität macht sich in einer Inaktiveriung der durch p53 induzierten Wachstumsinhibition bemerkbar. In Übereinstimmung dazu findet man in humanen Tumoren eine Überexpression von mdm2.

**Lit.:** Zaubermann et al.; Nucleic Acids Res. 23: 2584-2592, 1995.

### β2-Mikroglobulin

β2-Mikroglobulin wird auf allen kernhaltigen Zellen der Vertebraten exprimiert.

**Lit.:** Wiliams, A.F. and Barclay, A.N.; Annu. Rev. Immunol. 6, 381; 1988.

### MLH1

Synonyme: FCC2; COCA2; HNPCC (Hereditary Nonpolyposis colorectal cancer Type 2)

Mutationen MLH1-Gen sind für etwa 30% einer erblichen Form des Colonkarzinoms (hereditary nonpolyposis colon cancer = HNPCC) verantwortlich. Etwa 60% der HNPCC-Fälle werden aber durch Mutationen im MSH2-Gen auf Chromosom 2 verursacht. Es wurden zwei weitere humane Gene, die zu MutL homolog sind, isoliert: pms-1 und pms-2. Diese sind allerdings seltener als msh2 und mlhl an der Entstehung von Tumoren beteiligt.

**Lit.:** Bellacosa et al.; Am. J. Med. Genet. 62: 353-364, 1996.

### MMP (Metalloproteinase)

MMP's sind Zn²⁺-bindene Endopeptidasen, die Komponenten der extrazellulären Matrix abbauen. Sie sind an der Angiogenese und der Tumorinvasion beteiligt. Es existieren mind. 11 MMP's.

Eine Überexpression von Metalloproteinasen fördert die Invasion und Metastase von Tumoren. Einige dieser Metalloproteinasen werden von Tumorzellen sowohl auf der Zelloberfläche als auch auf mRNA-Ebene verstärkt exprimiert.

**Lit.:** Freije et al.; J. Biol. Chem. 269: 16766-16773, 1994; Sato et al.; Nature 370: 61-65, 1994.

### Motilin (MLN)

Motilin ist ein von Zellen des Dünndarms produziertes Hormon. **Lit.:** Daikh, D.I et al.; DNA 8: 615-621; 1989.

### MRP1 (Multidrug Resistance-Associated Protein-1)

Das MRP-Gen kodiert für eine in der Plasmamembran lokalisierte Chemotherapeutika-Efflux-Pumpe mit Ähnlichkeiten zur "ATP-binding casette"-Superfamilie von Transportsystemen, zu der auch MDR1 und der "cystic fibrosis transmembran conductance regulator" gehört. In einer chemoresistenten Zellinie des kleinzelligen Lungenkarzinoms konnte eine MDR1-Überexpression, die auf eine genomische Amplifikation des Gens zurückzuführen war, nachgewiesen werden.

**Lit.:** Cole et al.; Science 258: 1650-1654, 1992.

### MSH2

Dieses Gen spielt bei Tumoren eine Rolle, die sich bei Patienten mit hereditary nonpolyposis colorectal cancer (HNPCC) entwickeln. MSH2-Mutationen fanden sich in 21% der betroffenen HNPCC-Familien.

**Lit.:** Fishel R., et al.; Science 266, 1403-1405, 1994.

### MUC1 (Mucin-1)

### Synonym: urinary

Das MUC1-Gen kodiert eine transmembranes Glykoprotein, das von Tumorzellen zum Schutz gegenüber cytotoxischen Immunzellen und um die Metastasierung voranzutreiben gebildet wird. MUC1 wird von normalen Geweben und Zellen, aber auch von malignen Zellen und Geweben synthetisiert. Z.B. zeigen Brustkrebs-, Pankreaskrebs- und Adenokarzinomzellen eine Überexpression des MUC1-Proteins; außerdem wird bei einigen Krebsarten eine tumorspezifische Splicevariante neben der "normalen" Variante detektiert.

**Lit.:** Weiss et al.; Int. J. Cancer 66: 55-59, 1996.

### Muc18

Muc18 kodiert für Glykorotein, dessen Expression auf fortgeschrittene primäre und metastasierende Melanome sowie auf Zellinien der neuroektodermalen Linie beschränkt ist. In etwa 80% der Melanome wird eine mRNA-Expression gefunden, wobei die Expression mit dem Metastasierungszustand der Zellen korreliert. Die Anwesenheit von Zellen im Blut, die diese mRNA exprimieren, ist ein guter Hinweis auf zirkulierende Tumorzellen eines fortgeschrittenen oder metastasierenden Melanoms.

**Lit.:** Lehmann et al.; Proc. Natl. Acad. Sci. USA 86:9891-9895, 1989.

### myc

Synonym: Proto-Onkogen homolog zum myelozytomatoesen Virus; c-myc

Die Amplifikation von c-myc wird in fortgeschrittenen und in aggressiven, primären Tumoren gefunden.

**Lit.:** Adams et al.; Proc. Natl. Acad. Sci. 80: 1982-1986, 1983.

### N-CoR

N-CoR stellt ein Co-Repressorprotein für den Retinsäurerezeptor β dar.

**Lit.:** Soderstrom et al.; Mol. Endocrinol. 11:682 (1997).

Neurotensin (NTS)

Neurotensin ist ein kleines in den catecholaminhaltigen Neuronen lokalisiertes Neuropeptid.

**Lit.:** Bean, A.J. et al.; Neurosience 50: 259-268, 1992.

### NF-1

Bei dem NF1-Gen handelt es sich um einen Tumorsuppressor-Gen. Das Produkt des Neurofibromatose-1-Gens ist das Neurofibromin oder NF1-GAP. In 10 Familien mit Neurofibromatose (Morbus von Recklinghausen) wurden NF-1-Mutationen gefunden.

**Lit.:** Marchuk D.A., et al.; Genomics 11, 931-940, 1991.

### NF-2

### Synonym: merlin

Defekte des NF-2-Tumorsuppressor-Gens wurden bei Neurofibromatose Typ II, einer erblichen malignen Erkrankung mit bilateralen Tumoren des 8. cranialen Nerven, Neurofibromen, Meningiomen, Gliomen oder Schwannomen und auch bei sporadischen Meningiomen, Schwannomen und darüber hinaus bei Melanomen und Mammakarzinomen gefunden.

**Lit.:** Trofatter et al.; Cell 72: 791-800, 1993.

### nm23

Das nm23-Hl-Gen stellt einen potentiellen Metastasen-suppressor dar. Die Expression verhält sich negativ proportional zur Ausbildung von Lymphknotenmetastasen.

**Lit.:** Royds JA., et al.; J. Natl. Cancer Inst. 85, 727-31, 1993.

### ÖR (Östrogen-Rezeptor)

### Synonym: ER

Neben seiner Funktion als wichtiger Regulator von Wachstum und Differenzierung der Brustdrüse und des weiblichen Reproduktionstraktes spielt der Östrogenrezeptor in der Entwicklung von Mammakarzinomen eine Rolle. Der Gehalt eines Tumors an Östrogen- und Progesteronrezeptor ist somit ein wichtiger prognostischer Marker für das Anschlagen der endokrinen Therapie.

Vom Östrogenrezeptor wurden verschiedene Splice-Varianten beschrieben, denen eine Funktion bei der Tumorentstehung bzw. Metastasierung zugeschrieben wird. So wurde unter anderem in Brustkrebszellinien und Tumoren eine Variante des Östrogenrezeptors gefunden, dem das Exon 5 fehlt.

**Lit.:** Greene et al.; Science 231: 1150-1154, 1986.

### P-Glykoprotein (MDR1)

Synonyme: PGY-1; MDR1; GP170 Doxorubicin Resistance Gene; Multidrug Resistance Gene

Das mdrl-Gen kodiert für eine in der apikalen Membran lokalisierte Efflux-Pumpe für Cytostatika. Bei der Behandlung von Tumoren mit Chemotherapeutika tritt oft eine "multi drug resistance" gegen viele, strukturell verschiedene Therapeutika simultan auf. Experimentell konnte unter dem Einfluß von Chemotherapeutika eine Amplifikation des mdrl-Lokus beobachtet werden. In Zellinien mit bestehender Chemoresistenz wurde eine gesteigerte Expression dieses Gens gefunden.

**Lit.:** Gros et al.; Cell 47: 371-380, 1986.

### p16

Synomyme: p16(INK4) oder CDKN2; MTS1

Mit P16 wird der Cyclin-abhängige Kinase-Inhibitor bezeichnet. Charakteristisch für mtsl sind Deletionen in einer Vielzahl von Tumoren. In Melanomen sind sowohl Deletionen als auch Mutationen dieses Gens entdeckt worden. Die Häufigkeit von Deletionen des CDKN2-Gens in Tumorzellen weist auf ein Tumorsuppressor-Gen hin.

**Lit.:** Stone S., et al.; Cancer Res. 55, 2988-2994, 1995.

### p21

Das P21 betrifft den humanen Cyclin-abhängigen Kinase-Inhibitor.

**Lit.:** Harper J.W., et al.; Cell 75 (4), 805-16, 1993.

### p53

Bei Menschen gehören Mutationen im p53-Gen zu den häufigsten genetischen Veränderungen in bösartigen Tumoren. In der Mehrzahl dieser Tumoren findet man den Verlust eines Allels des p53-Gens (Mammakarzinom (32-64%), Ovarialkarzinom (44-66%); Magenkarzinom (>60%); Blasenkarzinom (38-58%); Pankreaskarzinom (70%); Lungenkarzinom (20%); Prostatakarzinom (59%); Cervixkarzinom (50%)). Die Mutationen finden sich über die gesamte Länge des Proteins verteilt, wobei es eine Häufung in den Exonen 5 bis 8 gibt und hierbei noch einige Exone vielfach betroffen sind (Codons 175, 245, 248, 249, 273). Die Frequenz dieser Hotspotmutationen ist je nach Ursprungsorgan des Tumors unterschiedlich. Mutationen im Codon 175 finden sich z.B. in 6% der Mammakarzinome, 14% der Kolorektaltumore und 4% der Ovarialkarzinome. Es handelt es sich fast ausschließlich um Punktmutationen, die in einem weiten Bereich des Gens vorkommen.

**Lit.:** Levine A.J.; Nature 351, 453, 1991.

### PDGF (Platelet-derived growth factor)

Synonyme: FDGF; GDGF; GDGF-1; GDGF-2; GSM; MDF; MDGF; ODGF; T47D factor

PDGF ist ein lokaler, autokriner und parakriner, chemotaktisch wirkender Wachstumsfaktor, potenter Vasokonstriktor und Angiogenesefaktor.

**Lit.:** Westermark und Sorg: Biology of platelet-derived growth factor. Karger, Basel 1993.

### Peptid YY

Synonym: PYY

PYY wird endokrin von Zellen des Dünndarms, des Colons und des Pankreas synthetisiert.

**Lit.:** Hort, Y. et al.; Genomics 26: 77-83, 1995.

### Perforin-1

Synonyme: Cytolysini C9-related protein; pore-forming protein; PFP

Perförin-1 gehört zu einer Klasse von cytolytischen Proteinen, welche die Membranen von Zielzellen permeablisieren.

**Lit.:** Ojcius und Young; TIBS 16: 225-229, 1991.

### PR (Progesteron-Rezeptor)

### Synonym: PGR

Ein hoher Gehalt an Progesteron-Rezeptor in einem Mammakarzinom ist ein prognostischer Marker für das Ansprechen einer endokrinen Therapie und längeres Überleben. In Übereinstimmung damit hat Progesteron eine schützende Wirkung gegenüber Brustkrebs. Die Analyse des Progesteron-Rezeptors in Mammakarzinomen ist besonders interessant, da indirekt auch die Anwesenheit des Östrogen-Rezeptor bestimmt werden kann.

**Lit.:** Misrahi er al.; Biochem. Biophys. Res. Commun. 143: 740-748, 1987.

### PSM (Prostata-spezifisches Membranantigen)

Normale und neoplastische Prostatazellen exprimieren PSM. **Lit.:** Israeli et al.; Cancer Res. 53: 227-230, 1993.

### PSA (Prostate-specifisches Antigen)

### Synonym: APS

Der PSA-Spiegel wird in Radioimmunoassays zur Diagnose und Überwachung von Prostatakarzinomen gemessen.

**Lit.:** Lundwall et al.; FEBS Lett. 214: 317-322, 1987.

### Ras

Die zellulären Gene der ras-Gen-Familie werden nach den entsprechenden retroviralen Onkogenen bezeichnet. Ihre Namen sind: c-Harvey-ras (c-H-ras), c-Kirsten-ras (c-K-ras) und N-ras (in Neuroblastomen entdeckt).

Mutationen in den Kodons 12, 13 und 61 finden sich sowohl in soliden als auch hämopoetische Tumoren. RAS-Mutationen können häufig in Pankreas-, Schilddrüsen- und colorectalen Karzinomen nachgewiesen werden. Die Mehrzahl der Mutationen in colorectalen Tumoren sind G-A- Transsitionen, was Rückschlüsse auf alkylierende Agenzien zuläßt.

**Lit.:** Bos J.L.; Cancer Res., 49, 4682-9, 1989.

### RB (Retinoblastom)

Der Verlust oder die Inaktivierung des RB-Gens ist für die Entstehung von Retinoblastomen entscheidend. Der Verlust der Funktion des Gens in beiden Allelen führt zur Tumorentstehung. Mikrosatelliten und RFLP können bei familiären Retinoblastom für DNA-Diagnostik verwendet werden.

**Lit.:** Friend S.H., et al.; PNAS 84, (24) 9059-63, 1987.

### RET

Das RET-Onkogen ist häufig in papillären Schilddrüsenkarzinomen rearrangiert und mit einem anderen Gen rekombiniert. Bei Patienten mit multipler endokriner Neoplasie vom Typ MEN 2A als auch bei Patienten mit familiärem Schilddrüsenkarzinom (FMTC) wurden zu einem hohen Prozentsatz Keimbahnmutationen des RET-Onkogens nachgewiesen.

**Lit.:** Viglietto-G et al.; Oncogene 1995 Sep 21, 11(6): 1207-10.

### SCCA-1 (Squamous Cell Carcinoma Antigen-1)

Das Protein SCCA-1 wurde aus einem metastatischen Cervix- "Squamous-Cell"-Karzinom isoliert. SCCA-1 wird als Marker für Plattenepithelkarzinome insbesondere der Cervix, des Halses und Nackens, der Lunge und des Ösophagus benutzt, wobei die Menge des Antigens im Blut mit dem Verlauf korreliert.

**Lit.:** Schneider et al.; Proc. Natl. Acad. Sci. U.S.A. 92: 3147-3151, 1995.

### P-, L- und E-Selectin

Selectine sind transmembrane Glykoproteine, die auf verschiedenen Zelltypen exprimiert werden, wie Plättchen (P-Selectin); Leukozyten (L-Selectin) und Endothelzellen (E- und P-Selectin). Das Expressionsmuster der Selectine und deren Liganden auf Zellen läßt Rückschlüsse auf das Rezirkulationsverhalten der entsprechenden Zelle zu.

**Lit.:** Springer, T.A. et al.; Cell 76: 301-314, 1994.

### SF (Scatter-Faktor)

### Synonym: Hepatocyte growth factor (HGF)

SF wird hauptsächlich von mesenchymalen Zellen, Stroma und Fibroblasten exprimiert und ist ein potenter Angiogenese- und Motilitätsfaktor, der als autokriner Faktor von Tumorzellen ihre Invasivität und die Tumorgenese vorantreiben kann.

**Lit.:** Nakamura et al.; Nature 342: 440-443, 1989; Bellusci et al.; Oncogene 9: 1091-1099, 1994.

### SF-Rezeptor c-met

### Synonyme: Proto-Onkogen met

Dem Proto-Onkogen c-met kommt eine wichtige Rolle bei der Tumorentstehung zu.

**Lit.:** Park et al.; Proc. Natl. Acad. Sci. USA 84: 6379-6383, 1987.

### STATS (Signal transduction and activator of transcription 5)

STAT's sind eine Familie von Proteine, die sowohl Signaltransduktions-Funktion ausüben, als auch Transkriptionsaktivatoren sind.

Lit.: Darnell; PNAS 93: 6221-6224, 1996.

### Surfactant-Proteine

Synonyme: Surfactant Protein (SP)-A, -B, -C; -D Surfactant-Protein A wird z.B. nur von Alveolarepithelzellen Typ II und Clara-Zellen im Lungengewebe und SP-C exklusiv nur von Alveolarzellen Type II exprimiert. Es existieren mehrere Surfactant-Proteine (A1 und A2, B, C und D), deren Expression (z.B. mRNA) in metastatischen, mikrometastasischen, pulmonären und extrapulmonären Adenokarzinomen, nichtkleinzelligen Lungenkarzinomen und Mammakarzinomen beschrieben wurde.

**Lit.:** Betz et al.; Cancer Res. 55: 4283-4286, 1995.

### Telomerase

Telomerasen definieren die Enden von Chromosomen.

**Lit.:** Morin G.B., et al.; Nature 353, 454-456, 1991. Blackburn E.H.; Nature 350, 569-573, 1991.

### TGF-alpha (Transforming growth factor alpha)

Synonyme: MDGF-2 (milk-derived growth factor 2); TGF-1; TCGF (transformed cell growth factor)

TGF-alpha wird von einer großen Anzahl von Karzinomen und (durch virale oder zelluläre Onkogene) transformierten Zellinien exprimiert. Es kann als autokriner Wachstumsfaktor bei Ovarkarzinomen oder als hämatopoetischer Wachstumsfaktor wirken und bei der Vaskularisation von Tumorgewebe eine Rolle zu spielen.

**Lit.:** Derynck; Advances in Cancer Research 58: 27-52, 1992.

### TIMP (Tissue Inhibitors of metalloproteinases)

Die TIMP's gehören zu einer Familie von Inhibitoren, welche die Aktivität von Metalloproteinasen hemmen, damit der Gewebeauflösung entgegenwirken und auch die Invasion und Metastase von Karzinomzellen in das Gewebe mitbestimmen.

**Lit.:** Apte et al.; Genomics 19: 86-90, 1994.

### TNF-alpha (Tumor-Nekrose-Faktor-alpha)

Synonyme: Cachectin; Monocyte/Macrophage-derived TNF; cytotoxin (CTX); enodogenous pyrogen; TNF-a TNF-alpha übt einen direkten cytotoxischen und apoptotischen Effekt auf Tumorzellen aus.

**Lit.:** Wang et al.; Science 228: 149-154, 1985.

### TNF-R1 p55

### Synonyme: CD120a; cytotoxischer TNF-R

TNF-R1 steht für den humanen Tumor-Nekrosis-Faktor-Rezeptor 1 und vermittelt hauptsächlich die Cytotoxizität und Apoptose.

**Lit.:** Loetscher H., et al.; Cell 61, 351-59, 1990.

### TNF-R2 p75

Synonym: C120b; TNFBR

TNF-R2 vermittelt hauptsächlich die T-Zellaktivierung.

**Lit.:** Beltinger C.P.; Genomics 35, 94-100, 1996.

### Topoisomerase II

Synonyme: TOPO; TOP2A; Topoisomerase Alpha

DNA-Topoisomerasen sind ATP-abhängige Enzyme, die den topologischen Status der DNA kontrollieren. Eine Chemoresistenz kann in solchen Tumoren gefunden werden, in denen die Aktivität der Topoisomerase herabgesetzt ist, wie beispielsweise durch eine reduzierte Expression. Weiterhin wurde eine Mutation des Topoisomerase-Gens aus chemoresistenten Zellinien isoliert, die bewirkt, daß das Enzym nicht mehr durch das Chemotherapeutikum inhibiert wird.

**Lit.:** Hinds et al.; Cancer Research 51: 4729-4731, 1991.

### Translokationen und Rearrangements

### B- und T-Zellrezeptor-Rearrangements

Im Verlauf der Differenzierung von B-Zellen werden die Immunglobulin(Ig)-Gene und während der Reifung von T-Zellen wird der T-Zell-Rezeptor (TCR) rearrangiert. Findet sich im Blut eines Patienten eine Vermehrung eines T-Zell Klones, läßt sich hinter dem polyklonalen Hintergrund aller restlichen T-Lymphozyten diese identifizieren.

**Lit.:** Trainor et al.; Blood, 78, 192-196, 1991. Lehman et al.; Hematopathology, 103, 171-176, 1995.

### Translokation (14;18)

Diese Translokation ist die häufigste in humanen Lymphomen.

Sie findet sich in mehr als 80% der follikulären Lymphome, in ca. 20% der diffusen großzelligen Lymphome und in ca. 50% der adulten undifferenzierten Lymphome.

**Lit.:** Barker et al.; Blood, 83, 1079-1085, 1994.

### Translokation (9;22)

Synonyme: Philadelphia-Chromosom, BCR/ABL

Das Philadelphia-Chromosom kommt durch eine reziproke Translokation zwischen den Chromosomen 9 und 22 zustande, die bei ca. 90% der Patienten mit chronischer myeloischer Leukämie (CML) in den Tumorzellen zu beobachten ist. In geringerer Inzidenz findet sich dieses Rearrangement auch bei akuten lymphatischen Leukämien. Es resultieren nur 3 verschiedene mögliche Fusionstranskripte, die zum Nachweis einer CML bzw. "minimal residual disease" genutzt werden können.

Lit.: Maurer et al.; The Lancet, 337, 1055-1058, 1991

### Translokationen (2;13) und (1;13)

In 68% der alveolären Rhabdomyosarkome findet sich als eine spezifische cytogenetische Anomalität die Translokation (2;13), in welche die Gene der Transkriptionsfaktoren PAX3 (Chromosom 2) und FKHR (Chromosom 13) involviert sind. 14% der alveolären Rhabdomyosarkome weisen die Translokation (1;13) auf, wobei statt des PAX3-Gens auf Chromosom 2 das PAX7-Gen auf Chromosom 1 involviert ist.

**Lit.:** Sreekantaiah et al.; American J. Pathol., 144: 1121-1134, 1194.

### Translokation (x;18)

Diese Translokation findet sich in 91% aller Synovialsarkome. Beteiligt sind das SSX-Gen von Chromosom X und das SYT-Gen von Chromosom 18, was zu einem Fusionstranskript, das vermutlich eine Schlüsselposition bei der Tumorentwicklung einnimmt.

**Lit.:** Clark et al.; Nature Genetics, 7, 502-508, 1994.

### Translokation (12;16)

Bei dieser Translokation, die in 77% aller myxoiden Liposarkome vorkommt, sind die Gene des Transkriptionsfaktors CHOP (Chromosom 12) und das FUS-Gen (Chromosom 16), dessen Funktion bislang unbekannt ist, involviert.

**Lit.:** Rabbits et al.; Nature Genetics, 4, 175-180, 1993.

### Translokation (11;22)

Diese Translokation findet sich in 86% aller Ewing's Sarkome und hat die Bildung eines Fusionstranskriptes zur Folge, das aus dem EWS-Gen (Chromosom 22) und dem FLI-Gen (Chromosom 11) besteht.

**Lit.:** West et al.; J. Clin. Oncol., 15, 583-588, 1997.

### α-und β-Tubulin

Tubuline stellen die Monomerbestandteile der Mikrotubuli des Cytoskeletts dar.

**Lit.:** J. Biol. Chem. 272:2534 (1997).

### Tyrosinase

Die Tyrosinase ist ein Schlüsselenzym der Melanin-Synthese, das ausschließlich in Melanozyten und Melanomzellen exprimiert wird. Der Nachweis Tyrosinase-exprimierender Zellen im Blut weist daher auf das Vorhandensein zirkulierender Melanomzellen im Blut hin.

**Lit.:** Giebel et al.; Genomics 9. 435-445, 1991.

### UPA (Urokinase-Typ-Plasminogenaktivator) und

### PAI-1 (Plasminogenaktivator-Inhibitor-1)

UPA ist ein proteolytisches Enzym, dessen Expression mit einer gesteigerten Invasivität, tumorassoziierter Angiogenese und Metastasierung korreliert. Seine Aktivität wird durch einen Inhibitor (PAI-1) reguliert. Untersuchungen unter anderem am primären Mammakarzinom und Magenkarzinom haben gezeigt, daß hohe Level an UPA mit einer schlechten Prognose einhergehen. In Übereinstimmung damit findet sich in aggressiven Tumorzellen keine Expression von PAI-1. Das Gleichgeweicht von UPA und PAI-1 bildet also einen prognostischen Parameter für die Metastasierungs- und Angiogenesefähigkeit eines Tumors.

**Lit.:** Ito et al.; Virchows Arch. 427:487-497, 1996.

### VEGF (Vaskularer Endothelialer Wachstumsfaktor)

Synonyme: VPF; vascular permeability factor; vasculotropin; CD(glioma-derived)-VEGF

VEGF kann als Folge alternativen Splicens der mRNA in vier Formen auftreten, nämlich VEGF121 und VEGF165 sowie VEGF189 und VEGF206. VEGF scheint eine wichtige Rolle in der Kontrolle der Blutgefäßformation und -permeabilität zu spielen und darüber hinaus ein Hauptregulator der Tumorangiogenese zu sein.

**Lit.:** Leung et al.; Science 246: 1306-1309, 1989. Tischer et al.; J. Biol. Chem. 266: 11947-11954, 1991.

### VEGF-R1 (VEGF-Rezeptor 1)

Synonyme: FMS-like Tyrosine kinase-1; flt1 vascular endothelial growth factor/vascular permeability factor receptor; oncogene flt

Vielfach wird eine erhöhte Expression von VEGF-R1 in Karzinomen beschrieben.

**Lit.:** Shibuya et al.; Oncogene 5: 519-524, 1990.

### VEGF-R2 (VEGF-Rezeptor-2)

Synonyme: KDR; Tyrosine kinase growth factor receptor; FLK-1 receptor for vascular endothelial growth factor; FLK1; kinase insert domain receptor Die Hochregulation von VEGF-mRNA in Tumorzellen und der mRNA seiner Rezeptoren in der Tumorvaskulatur korreliert mit einer erhöhten Aggressivität des Tumors. VEGF-R2 wird außerdem in Melamom- und Ovartumorzellen transkribiert.

**Lit.:** Terman et al.; Oncogene 6: 1677-1683, 1991; Boocock et al.; J. Natl. Cancer Inst. 87: 506-516, 1995.

### VHL (Von Hippel-Lindau Syndrom)

Das VHL-Genprodukt ist ein Tumorsuppressor-Protein, das bei erblichen Mutationen zu Nierenkarzinomen, Hämangiomen von Kleinhirn und Retina, Phäochromocytomen und Ependymomen führt. Eine Beteiligung an der Entstehung spontaner Tumoren konnte ebenfalls gezeigt werden.

**Lit.:** Latif et al.; Science 260: 1317-1320, 1993.

### Virale Onkogene

Von besonderer Bedeutung für die virale Onkogenese sind Hepatitis-B- und -C-Viren, gegebenenfalls auch SV40, in Bezug auf Leberzellkarzinome, das HTLV-1-Virus im Zusammenhang mit T-Zell-Lymphomen, das Epstein Barr Virus (EBV) im Zusammenhang mit Burkitt-Lymphomen, Nasopharyngial-Karzinomen und der Hodgkin-Erkrankung und humane Papillomaviren der Typen 16 und 18 im Zusammenhang mit Karzinomen im Urethro-Genital Bereich, insbesondere der Cervix.

Weiterhin werden Herpesviren der Typen 4 und 6 und das HI-Virus im Zusammenhang mit der Entstehung von Tumoren diskutiert.

**Lit.:** Mueller M.; Environ. Health Perspect. 103 (1995) Suppl.8, 259-261.

### Leberzellkarzinome

**Lit.:** De-Vita S. et al.; Blood 86 (1995) 1887-1892. Koike K.; Intervirology 38 (1995) 134-142.

Casola S. et al.; Acta Genet. Med. Gemellol Roma 45 (1996) 221-225.

E Hara et al.; Dev.Genet. 18 (1996) 161-172.

### Urethro-Genitial-Karzinome (Cervixkarzinome,

Zervikalkarzinome, Vulvakarzinome, Prostatakrebs, Analkrebs, Blasenkrebs)

Lit.: Beyer-Finkler E. et al.; Intervirology 38 (1995) 173-180.

Moyet-Lalle C. et al.; Int. J. Cancer 64 (1995) 124-129.

### Lymphome (Non-Hodgkin-Lymphome, Burkitt-Lymphome, T-Zell-Lymphome, B-Zell-Lymphome)

**Lit.:** Lee J.H. et al.; J. Korean Med. Sci. 10 (1995) 399-405; Tomita Y. et al.; Leuk. Lymphöma 19 (1995) 129-134.

Wang C.Y. et al.; Mayo Clin. Proc. 70 (1995) 665-672; Maroushek S.R. et al.; Microb. Pathog. 19 (1995) 317-333.

### Lungenkarzinome

**Lit.:** Hogg J.C., Hegele R.G.; Semin Resp. Infect. 10 (1995) 244-253.

### WT1

Bei den Wilms-Tumoren handelt es sich um Nierentumore des Kindes. Es handelt sich mit großer Wahrscheinlichkeit um ein Tumorsuppressorgen.

Lit.: Bonetta L., et al.; Cytogenet. Cell Genet. 51, 1989. Gessler M., et al.; Genomics 12, 807-813, 1992.

## Patentansprüche

1. Verfahren zur Charakterisierung disseminierter und mikrometastasierter Krebszellen mittels Nukleinsäureanalyse, wobei man
i) aus Körperflüssigkeit eines Individuums gewonnene Zellen auf wenigstens eine mRNA eines krebsspezifischen ersten Gens untersucht; und
ii) aus Körperflüssigkeit eines Individuums abgetrennte Krebszellen auf wenigstens eine DNA oder mRNA eines krebsspezifischen oder krebsassoziierten zweiten Gens untersucht und die gleiche Untersuchung mit Nichtkrebszellen desselben Individuums zum Vergleich durchführt,
wobei das erste und das zweite Gen verschieden sind.

2. Verfahren nach Anspruch 1, **dadurch gekennzeichnet, dass** die Körperflüssigkeit Blut oder Knochenmark ist.

3. Verfahren nach Anspruch 1 oder 2, **dadurch gekennzeichnet, dass** die aus Körperflüssigkeit des Individuums gewonnenen Zellen erhältlich sind, indem man Zellen, ein zellhaltiges Konzentrat oder eine zellhaltige Flüssigkeit der Körperflüssigkeit gewinnt.

4. Verfahren nach Anspruch 3, **dadurch gekennzeichnet, dass** die Zellen, das zellhaltige Konzentrat oder die zellhaltige Flüssigkeit eine mononukleäre Zellen umfassende Fraktion aus Blut ist.

5. Verfahren nach einem der Ansprüche 1 bis 4, **dadurch gekennzeichnet, dass** die aus Körperflüssigkeit eines Individuums abgetrennten Krebszellen erhältlich sind, indem man Krebszellen aus der Körperflüssigkeit oder aus zellhaltigen Fraktionen davon durch Immunadsorption, Mikro-Filtration oder Dichtegradientenzentrifugation abtrennt.

6. Verfahren nach einem der Ansprüche 1 bis 5, **dadurch gekennzeichnet, dass** die Untersuchung der aus Körperflüssigkeit des Individuums gewonnenen Zellen auf wenigstens eine mRNA eines krebsspezifischen ersten Gens eine Expressionsanalyse der mRNA beinhaltet, wobei das Gen von Nichtkrebszellen der untersuchten Körperflüssigkeit im Wesentlichen nicht exprimiert wird.

7. Verfahren nach Anspruch 6, **dadurch gekennzeichnet, dass** die mRNA eine von dem Gen CEA, CK20, MUC1, Tyrosinase oder MAGE3 exprimierte mRNA ist.

8. Verfahren nach einem der Ansprüche 1 bis 7, **dadurch gekennzeichnet, dass** die Untersuchung der aus Körperflüssigkeit des Individuums abgetrennten Krebszellen auf wenigstens eine DNA oder mRNA eines krebsspezifischen oder krebsassoziierten zweiten Gens eine Untersuchung auf Mutationen, Amplifikationen, LOHs, Translokationen oder Polymorphismen von genomischer DNA beinhaltet.

9. Verfahren nach Anspruch 8, **dadurch gekennzeichnet, dass** die genomische DNA eine genomische DNA des Gens p53, erb-B2, c-myc, K-ras, RB, APC oder DCC ist.

10. Verfahren nach einem der Ansprüche 1 bis 9, **dadurch gekennzeichnet, dass** die Untersuchung der aus Körperflüssigkeit des Individuums abgetrennten Krebszellen auf wenigstens eine DNA oder mRNA eines krebsspezifischen oder krebsassoziierten zweiten Gens eine Untersuchung auf im Vergleich zu Nichtkrebszellen der Körperflüssigkeit desselben Individuums modulierte Expression des Gens beinhaltet.

11. Verfahren nach Anspruch 10, **dadurch gekennzeichnet, dass** das Gen von Nichtkrebszellen der Körperflüssigkeit desselben Individuums exprimiert wird.

12. Verfahren nach einem der Ansprüche 1 bis 11, **dadurch gekennzeichnet, dass** das krebsassoziierte Gen ein gewebsspezifisches Gen, metastasierungsassoziiertes Gen, Steroidhormonrezeptor-Gen, Chemoresistenz-Gen, oder Gen, das mit der Immunmodulation, Zellproliferation oder Apoptose korreliert, ist.

13. Verfahren nach Anspruch 12, **dadurch gekennzeichnet, dass** das metastasierungsassoziierte Gen für einen Angiogenesefaktor, Motilitätsfaktor, Wachstumsfaktor, Matrixdegradationsfaktor oder Adhäsionsfaktor kodiert.

14. Verfahren nach einem der Ansprüche 10 bis 13, **dadurch gekennzeichnet, dass** die mRNA eine von dem Gen bFGF, bFGF-R, VEGF, VEGF-R1, VEGF-R2 MMP2 oder TIMP3 exprimierte mRNA ist.

15. Verwendung des Verfahrens nach einem der Ansprüche 1 bis 14 zur in vitro-Diagnose von Krebs.

16. Verwendung des Verfahrens nach einem der Ansprüche 1 bis 14 zum Nachweis disseminierter und mikrometastasierter Krebszellen.

17. Verwendung des Verfahrens nach einem der Ansprüche 1 bis 14 zur Organlokalisation der Streuquelle disseminierter und mikrometastasierter Krebszellen oder zur Erstellung eines Risikoprofils disseminierter und mikrometastasierter Krebszellen.

## Claims

1. Method for the characterization of disseminated and micrometastasized cancer cells by nucleic acid analysis, which comprises
i) investigating cells obtained from body fluid of an individual for at least one mRNA of a cancer-specific first gene; and
ii) investigating cancer cells removed from body fluid of an individual for at least one DNA and/or mRNA of a cancer-specific or cancer-associated second gene and carrying out the same investigation with non-cancer cells from the same individual for comparison,
the first and second gene being different from each other.

2. Method according to claim 1, **characterized in that** the body fluid is blood or bone marrow.

3. Method according to claim 1 or 2, **characterized in that** the cancer cells obtained from body fluid of the individual are obtainable by preparing cells, a cell-containing concentrate or cell-containing fluid of the body fluid.

4. Method according to claim 3, **characterized in that** the cells, the cell-containing concentrate or the cell-containing fluid is a blood fraction comprising mononuclear cells.

5. Method according to any one of claims 1 to 4, **characterized in that** the cancer cells removed from body fluid of the individual are obtainable by separating cancer cells from the body fluid or from cell-containing fractions thereof by immunoadsorption, microfiltration or density gradient centrifugation.

6. Method according to any one of claims 1 to 5, **characterized in that** the investigation of the cells obtained from body fluid of the individual for at least one mRNA of a cancer-specific first gene comprises an expression analysis of the mRNA, the gene being essentially not expressed in non-cancer cells in the body fluid investigated.

7. Method according to claim 6, **characterized in that** the mRNA is an mRNA expressed by the gene CEA, CK20, MUC1, tyrosinase or MAGE3.

8. Method according to any one of claims 1 to 7, **characterized in that** the investigation of the cells removed from body fluid of the individual for at least one DNA or mRNA of a cancer-specific or cancer-associated second gene comprises an investigation of mutations, amplifications, LOHs, translocations and/or polymorphisms of genomic DNA.

9. Method according to claim 8, **characterized in that** the genomic DNA is a genomic DNA of the gene p53, erb-B2, c-myc, K-ras, RB, APC or DCC.

10. Method according to any one of claims 1 to 9, **characterized in that** the investigation of the cancer cells removed from body fluid of the individual for at least one DNA or mRNA of a cancer-specific or cancer-associated second gene comprises an investigation for an expression of the gene that is modulated when compared to non-cancer cells in the body fluid of the same individual.

11. Method according to claim 10, **characterized in that** the gene is expressed by non-cancer cells in the body fluid of the same individual.

12. Method according to any one of claims 1 to 11, **characterized in that** the cancer-associated gene is a tissue-specific gene, metastasis-associated gene, steroid hormone receptor gene, drug resistance gene or gene which correlates with immunomodulation, cell proliferation or apoptosis.

13. Method according to claim 12, **characterized in that** the metastasis-associated gene codes for an angiogenesis factor, motility factor, growth factor, matrix degradation factor or adhesion factor.

14. Method according to any one of claims 10 to 13, **characterized in that** the mRNA is an mRNA expressed by the gene bFGF, bFGF-R, VEGF, VEGF-R1, VEGF-R2, MMP2 or TIMP3.

15. The use of the method according to any one of claims 1 to 14 for the *in vitro-*diagnosis of cancer.

16. The use of the method according to any one of claims 1 to 14 for detecting disseminated and micrometastasized cancer cells.

17. The use of the method according to any one of claims 1 to 14 for localizing the source of spread of disseminated and micrometastasized cancer cells to an organ or drawing up a risk profile of disseminated and micrometastasized cancer cells.

## Revendications

1. Procédé de caractérisation de cellules cancéreuses disséminées et micrométastasées, au moyen d'une analyse d'acide nucléique, selon lequel
i) on examine des cellules obtenues à partir d'un liquide du corps d'un individu, afin de détecter au moins un ARNm d'un premier gène spécifique d'un cancer; et
ii) on examine des cellules cancéreuses séparées d'un liquide du corps d'un individu, afin de détecter au moins un ADN ou un ARNm d'un deuxième gène spécifique d'un cancer ou associé à un cancer, et on effectue le même examen avec des cellules non cancéreuses du même individu, à des fms de comparaison,
le premier gène et le deuxième gène étant différents.

2. Procédé selon la revendication 1, **caractérisé par le fait que** le liquide du corps est du sang ou de la moelle osseuse.

3. Procédé selon la revendication 1 ou 2, **caractérisé par le fait que** les cellules obtenues à partir d'un liquide du corps de l'individu peuvent être obtenues en obtenant des cellules, un concentré contenant des cellules ou un liquide, contenant des cellules, du liquide du corps.

4. Procédé selon la revendication 3, **caractérisé par le fait que** les cellules, le concentré contenant des cellules ou le liquide contenant des cellules sont une fraction de sang comportant des cellules mononucléaires.

5. Procédé selon une des revendications 1 à 4, **caractérisé par le fait que** les cellules cancéreuses séparées d'un liquide du corps d'un individu peuvent être obtenues en séparant des cellules cancéreuses du liquide du corps ou de fractions de ce liquide, contenant des cellules, par immunoadsorption, microfiltration ou centrifugation sur gradient de densité.

6. Procédé selon une des revendications 1 à 5, **caractérisé par le fait que** l'examen des cellules obtenues à partir d'un liquide du corps de l'individu, afin de détecter au moins un ARNm d'un premier gène spécifique d'un cancer, comprend une analyse d'expression de l'ARNm, le gène n'étant sensiblement pas exprimé par des cellules non cancéreuses du liquide de corps examiné.

7. Procédé selon la revendication 6, **caractérisé par le fait que** l'ARNm est un ARNm exprimé par le gène CEA, CK20, MUC1, tyrosinase ou MAGE3.

8. Procédé selon une des revendications 1 à 7, **caractérisé par le fait que** l'examen des cellules cancéreuses séparées d'un liquide du corps de l'individu, afin de détecter au moins un ADN ou un ARNm d'un deuxième gène spécifique d'un cancer ou associé à un cancer, comprend un examen pour déceler des mutations, des amplifications, des LOH, des translocations ou des polymorphismes d'ADN génomique.

9. Procédé selon la revendication 8, **caractérisé par le fait que** l'ADN génomique est un ADN génomique du gène p53, erb-B2, c-myc, K-ras, RB, APC ou DCC.

10. Procédé selon une des revendications 1 à 9, **caractérisé par le fait que** l'examen des cellules cancéreuses séparées d'un liquide du corps de l'individu, afin de détecter au moins un ADN ou un ARNm d'un deuxième gène spécifique d'un cancer ou associé à un cancer, comprend un examen pour déceler une expression du gène qui est modulée par rapport à des cellules non cancéreuses du liquide du corps du même individu.

11. Procédé selon la revendication 10, **caractérisé par le fait que** le gène est exprimé par des cellules non cancéreuses du liquide du corps du même individu.

12. Procédé selon une des revendications 1 à 11, **caractérisé par le fait que** le gène associé à un cancer est un gène spécifique d'un tissu, un gène associé à une diffusion métastatique, un gène récepteur des hormones stéroïdes, un gène de chimiorésistance ou un gène qui est en corrélation avec l'immunomodulation, la prolifération cellulaire ou l'apoptose.

13. Procédé selon la revendication 12, **caractérisé par le fait que** le gène associé à une diffusion métastatique code pour un facteur d'angiogénèse, un facteur de motilité, un facteur de croissance, un facteur de dégradation matricielle ou un facteur d'adhésion.

14. Procédé selon une des revendications 10 à 13, **caractérisé par le fait que** l'ARNm est un ARNm exprimé par le gène bFGF, bFGF-R, VEGF, VEGF-R1, VEGF-R2 MMP2 ou TIMP3.

15. Utilisation du procédé selon une des revendications 1 à 14, pour effectuer un diagnostic in vitro d'un cancer.

16. Utilisation du procédé selon une des revendications 1 à 14, pour déceler des cellules cancéreuses disséminées et micrométastasées.

17. Utilisation du procédé selon une des revendications 1 à 14, pour effectuer la localisation organique de la source de diffusion de cellules cancéreuses disséminées et micrométastasées ou pour établir un profil des risques de cellules cancéreuses disséminées et micrométastasées.
